# EUROPEAN PATENT APPLICATION

(11) **EP 4 592 381 A2**
(43) Date of publication of application: **30.07.2025**
(21) Application number: 25165176.6
(22) Date of filing: 27.07.2020
(51) Int. Cl.: C12N 5/07

(54) **BIOENGINEERED FORMULATION, PROCESS FOR PREPARING AND IMPLEMENTATIONS THEREOF**

(30) Priority: 26.07.2019 IN 201941030371
(62) Divisional of application: 20846372.9
(71) Applicant: Pandorum Technologies Private Limited, Bengaluru 560065 (IN)
(72) Inventor: BHOWMICK, Tuhin, 560065 Bengaluru (IN); CHANDRU, Arun, 560065 Bengaluru (IN); SELVAM, Shivaram, 560065 Bengaluru (IN); AGRAWAL, Parinita, 560065 Bengaluru (IN); BEN THOMAS, Midhun, 560065 Bengaluru (IN); S, Kamalnath, 560065 Bengaluru (IN); MENON, Deepthi, 560065 Bengaluru (IN)
(74) Representative: Cooley (UK) LLP

(57) **Abstract**

The present disclosure discloses a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%. The present disclosure also discloses the bioengineered formulation comprising stem cells, or exosomes, or combinations thereof. Further, the process for preparing the bioengineered formulation is also disclosed therein. Moreover, the formulation comprising: a) exosomes selected from the group consisting of corneal stromal stem cell derived-exosomes, primed mesenchymal stem cell derived-exosomes, and naive mesenchymal stem cell derived-exosomes; and (b) a clinically approved eye drop formulation is also provided.

## Description

### FIELD OF INVENTION

The present disclosure broadly relates to biological hydrogel formulation. Particularly, the present disclosure relates to a bioengineered formulation for corneal applications. The present disclosure also provides a process for preparing the bioengineered formulation, and applications thereof.

### BACKGROUND OF INVENTION

Corneal blindness is the fourth leading cause of blindness in the world and an estimated 1.5 million new cases have been reported worldwide each year. About 10 million people in the world are affected by bilateral corneal blindness and another 23 million with unilateral corneal blindness. The leading causes of corneal dysfunction include trachoma (involving scarring and vascularization of the cornea), ocular trauma, corneal ulceration, and infections, such as those due to herpes simplex virus (Corneal blindness: a global perspective. Whitcher JP, Srinivasan M, Upadhyay MP Bull World Health Organ. 2001; 79(3):214-21). One of the key medical treatments for corneal diseases include keratoplasty (corneal transplant). However, there are various complications associated with cornea transplant, which includes: (i) keratoplasty patients experiences organ (cornea) rejection; (ii) scarring from infections, such as eye herpes or fungal keratitis; (iii) glaucoma (increased pressure inside the eye); (iv) visual acuity problems (sharpness of the vision) caused by an irregular curve in the shape of the cornea; (v) detachment of the corneal transplant; (vi) high cost and inconveniences surrounding the safe extraction, storage, and transportation of living tissue.

Seeing the limitations associated with cornea transplant, various efforts have been made by the scientists. In the recent years, the use of biomaterials and the incorporation of recipient's own cells in tissue engineering have become a paramount importance to resolve the issues associated with cornea transplant. For instance, tissue adhesives have been extensively used for closure of ocular wounds after an injury or during corneal surgeries. In corneal surgeries, they are primarily employed as suture-less substitutes for closing perforations post-surgery. Various biomaterials are reported in the literal for treating eye diseases. For instance, JP2014129408A discloses a biomaterial comprising treated chitosan, modified chitosan, modified treated chitosan, or a mixture or combination thereof, wherein at least one chitosan is treated chitosan, modified chitosan, or modified treated chitosan. The method for making the biomaterial and using the same is also disclosed in the document.

However, conventional ocular adhesives are plagued with notable disadvantages including, rapid polymerization and heat generation, low biocompatibility, low transparency and rough surfaces, difficulty in handling, short residence times and poor integration with host ocular tissues.

Thus, there exists a long-felt need in the art to develop an efficient, biocompatible and biodegradable cross-linked hydrogel formulation to match the characteristics of the native cornea that would help in treating the corneal diseases avoiding any side effects.

### SUMMARY OF THE INVENTION

In an aspect of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%.

In another aspect of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the bioengineered formulation has a compressive modulus in the range of 100-1400 kPa, preferably 100-500 kPa.

In another aspect of the present disclosure, there is provide a process for obtaining a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%, said process comprising: (i) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix; and (ii) contacting the pre-mix with a photo-initiator solution, to obtain the bioengineered formulation.

In another aspect of the present disclosure, there is provided a method for treating a corneal defect in a subject, said method comprises: (a) obtaining the bioengineered formulation as described herein; (b) applying a suitable amount of the bioengineered formulation at the site of a corneal defect; and (c) illuminating a white light having an intensity in the range of 50-150mW/cm² on the formulation at the site of the corneal defect for a time period in a range of 1-15 minutes, preferably, 2-8 minutes, for treating the corneal defect in a subject.

In another aspect of the present disclosure, there is provided a formulation comprising: (a) exosomes selected from the group consisting of corneal stromal stem cell derived-exosomes, primed mesenchymal stem cell derived-exosomes, and naive mesenchymal stem cell derived-exosomes; and (b) a clinically approved eye drop formulation.

In another aspect of the present disclosure, there is provided a method for treating a corneal defect in a subject, said method comprising: (a) obtaining a formulation comprising: (i) exosomes selected from the group consisting of corneal stromal stem cell derived-exosomes, primed mesenchymal stem cell derived-exosomes, and naive mesenchymal stem cell derived-exosomes; and (ii) a clinically approved eye drop formulation; and (b) applying the formulation at the site of the corneal defect, for treating the corneal defect in a subject.

These and other features, aspects, and advantages of the present subject matter will be better understood with reference to the following description and appended claims. This summary is provided to introduce a selection of concepts in a simplified form. This summary is not intended to identify key features or essential features of the claimed subject matter, nor is it intended to be used to limit the scope of the claimed subject matter.

### BRIEF DESCRIPTION OF ACCOMPANYING DRAWINGS

The following drawings form a part of the present specification and are included to further illustrate aspects of the present disclosure. The disclosure may be better understood by reference to the drawings in combination with the detailed description of the specific embodiments presented herein.

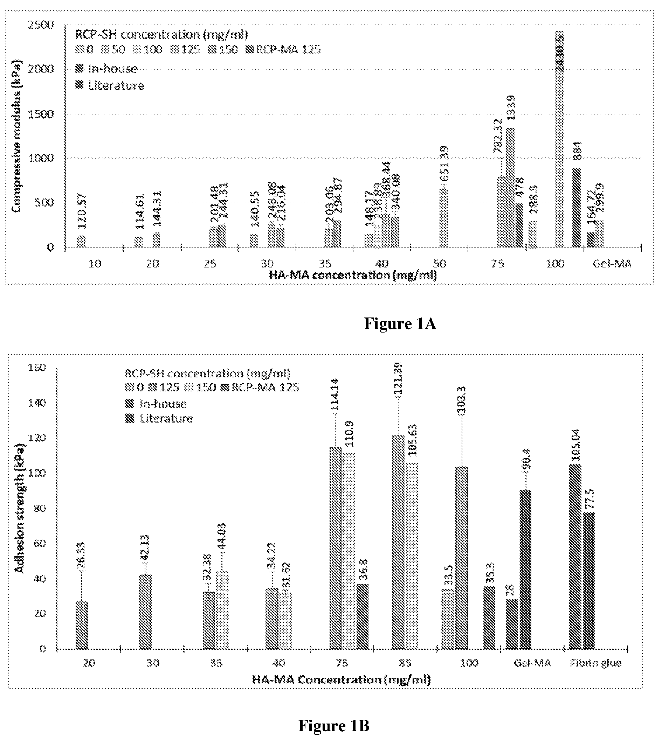

Figure 1 depicts the graphical representation of the screening results of the bioengineered formulations comprising "33 kDa" HA-MA/RCP-SH (both with degree of substitution (DoS) 50%) for: (A) Compressive Modulus and (B) Adhesion Strength. Bar graph (mean±SD, n=3), in accordance with an embodiment of the present disclosure.

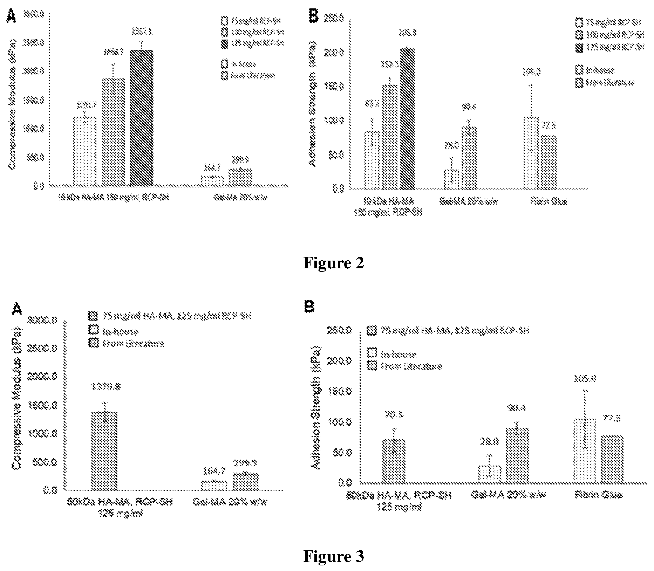

Figure 2 depicts the graphical representation of the screening results of the bioengineered formulations comprising 10 kDa HA-MA/RCP-SH (both with degree of substitution (DoS) 30%) for: (A) Compressive Modulus and (B) Adhesion Strength. Bar graph (mean +/- SD, n=3), in accordance with an embodiment of the present disclosure.
Figure 3 depicts the graphical representation of the screening results of the bioengineered formulations comprising 50 kDa HA-MA/RCP-SH (both with degree of substitution (DoS) 50%) for: (A) Compressive Modulus and (B) Adhesion Strength. Bar graph (mean ± SD, n=3), in accordance with an embodiment of the present disclosure.

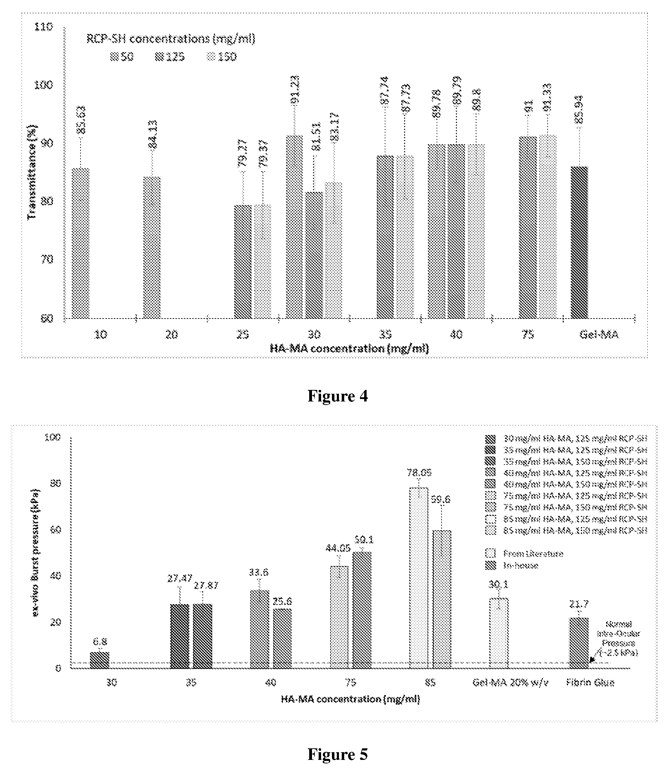

Figure 4 depicts visible light transmittance-based screening for "33 kDa" HA-MA/RCP-SH formulations (both DoS 50%) with respect to 1X PBS shown in comparison with Gel-MA. Bar graph (mean +/- SD, n=3), in accordance with an embodiment of the present disclosure.
Figure 5 depicts the *ex-vivo* burst pressure testing for "33 kDa" HA-MA/RCP-SH and Gel-MA (20% w/w, DoS >95%) formulations. Bar graph (mean ± SD, n=3). Target value is set at 2.5kPa, in accordance with an embodiment of the present disclosure.

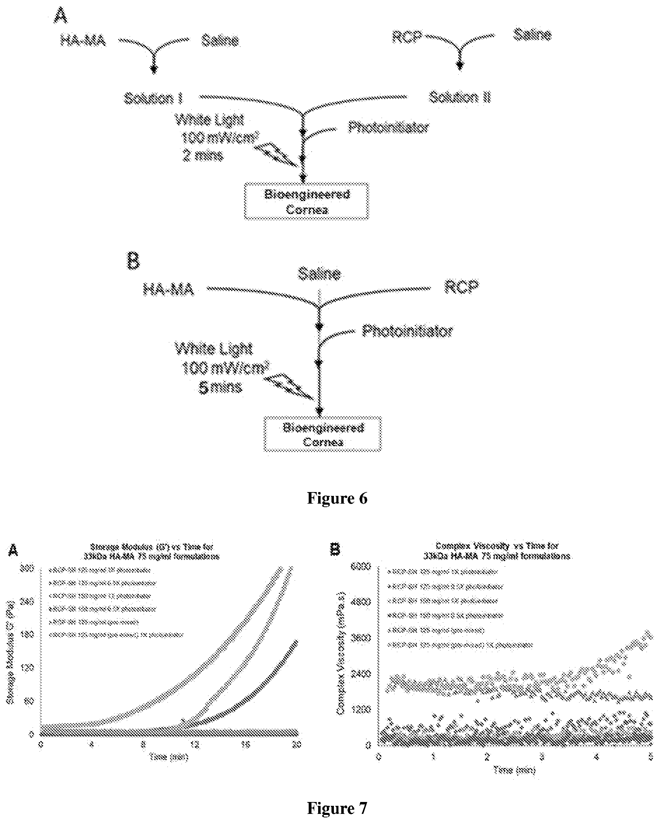

Figure 6 depicts: (A) Bioengineered formulation preparation protocol where the components are separately reconstituted and mixed (B) Pre-mixed Bioengineered formulation preparation modified protocol where the components are pre-mixed in powder form and then reconstituted in saline solution, in accordance with an embodiment of the present disclosure.
Figure 7 depicts the cross-linking kinetics for "33 kDa" HA-MA/RCP-SH formulations with DoS 50% in ambient light with plots for (A) storage modulus G' and **(B)** complex viscosity, in accordance with an embodiment of the present disclosure.

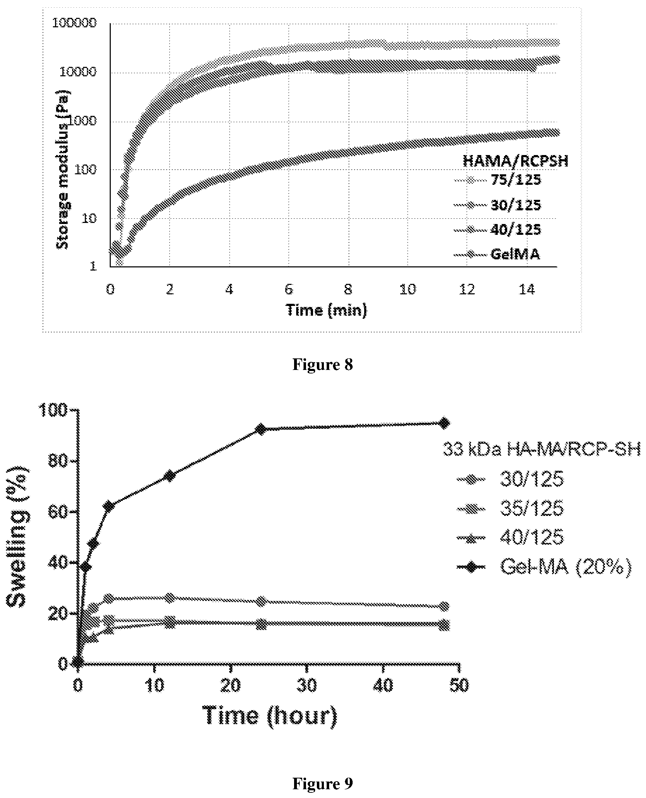

Figure 8 depicts the comparison of the crosslinking kinetics of pre-mixed HA-MA/RCP-SH (mg/ml, DoS 50%) formulations with the crosslinking kinetics of Gel-MA (20% w/w, DoS >95%) at 100 mW/cm² white light, in accordance with an embodiment of the present disclosure.
Figure 9 depicts the swelling profile of "33 kDa" HA-MA/RCP-SH hydrogel formulations with DoS 50% compared to Gel-MA (20% w/w, DoS >95%) with respect to time (error bars represent standard deviation for n=3 samples), in accordance with an embodiment of the present disclosure.

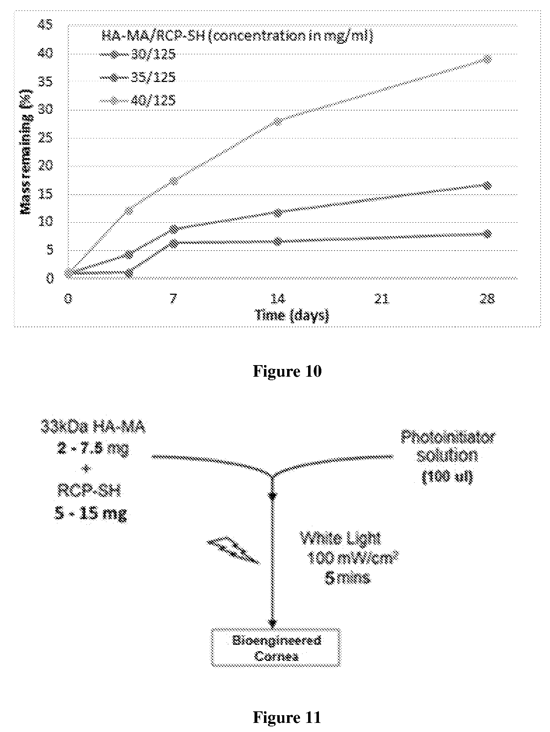

Figure 10 depicts the biodegradation profile of "33 kDa" HA-MA/RCP-SH hydrogel formulations (with DoS 50%) compared with respect to time, in accordance with an embodiment of the present disclosure.
Figure 11 depicts the optimized protocol for preparing the bioengineered formulation of the present disclosure, in accordance with an embodiment of the present disclosure.

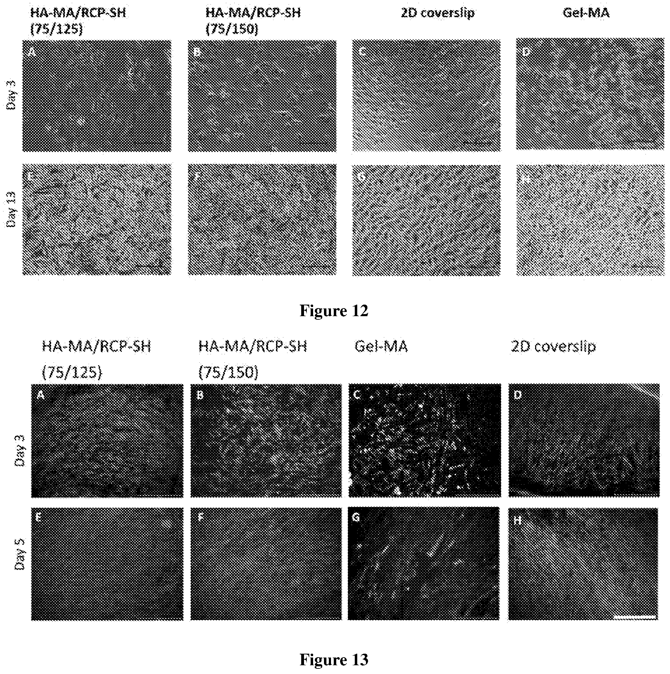

Figure 12 depicts phase contrast microscopy image depicting epithelialization of the 2D coverslip, Gel-MA (20%, DoS >95%), HA-MA/RCP-SH (mg/ml, DoS 50%) hydrogel surface with primary human corneal epithelial cells on day 3 and 13 *in vitro.* (Scale bar = 100 µm), in accordance with an embodiment of the present disclosure.
Figure 13 depicts the cell viability study for CLSCs cultured on the 'Bioengineered Cornea' HA-MA/RCP-SH hydrogel surface (mg/ml, DoS 50%) and Gel-MA (20%, DoS >95%) and 2D culture surfaces. (Scale bar= 500 µm), in accordance with an embodiment of the present disclosure.

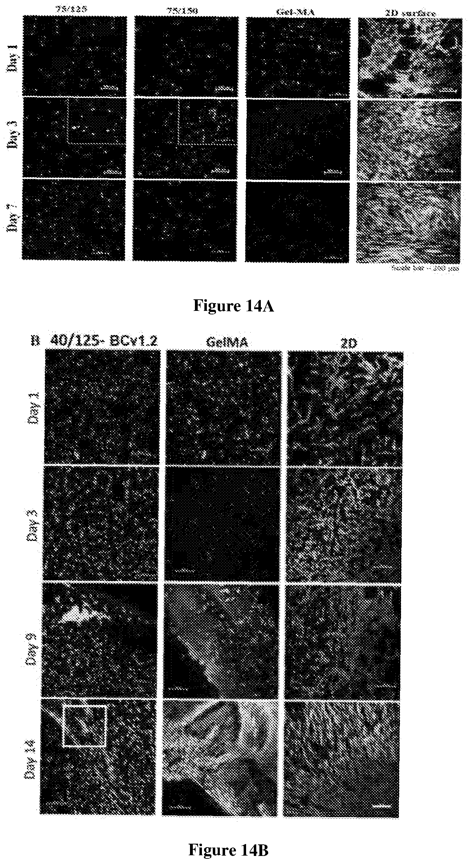

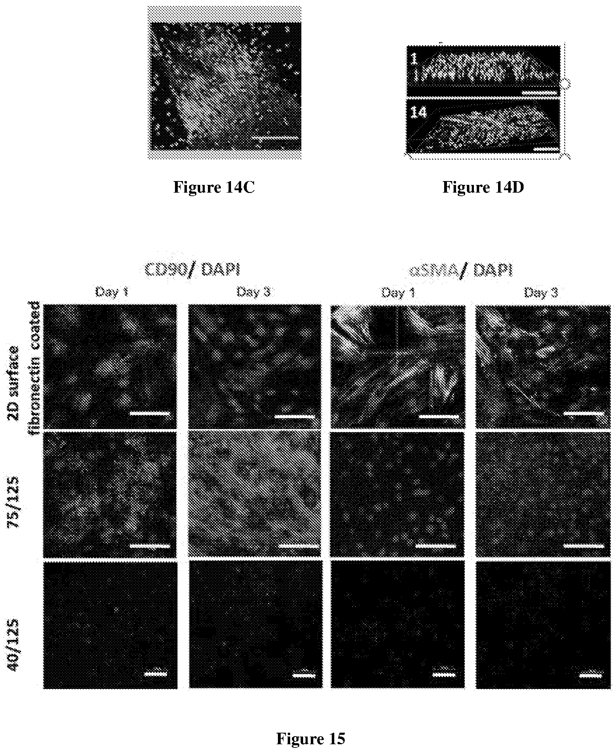

Figure 14 depicts cell viability study for the CLSCs encapsulated in the hydrogel formulations of the present disclosure and Gel-MA (20%, DoS >95%). Cells on coverslips were cultured on the surface. **A)** and **B)** represent experiments performed on different HA-MA and RCP-SH formulations (mg/ml, DoS 50%), Gel-MA and 2D surface. **C)** depicts enlarged image of the area marked in day 14 for 40/125 (mg/ml, DoS 50%) BCv1.2 formulation. **D)** depicts layer-by-layer 3D construct of images depicting homogenous distribution of live cells within the BCv1.2 hydrogel. (Scale bar= 200 µm), in accordance with an embodiment of the present disclosure.
Figure 15 depicts immunofluorescence study showing expression of CD90 (presented in red color) and αSMA (presented in green color) by the CLSCs encapsulated in the hydrogel formulation with respect to 2D culture surface. Scale bar = 100 µm, in accordance with an embodiment of the present disclosure.

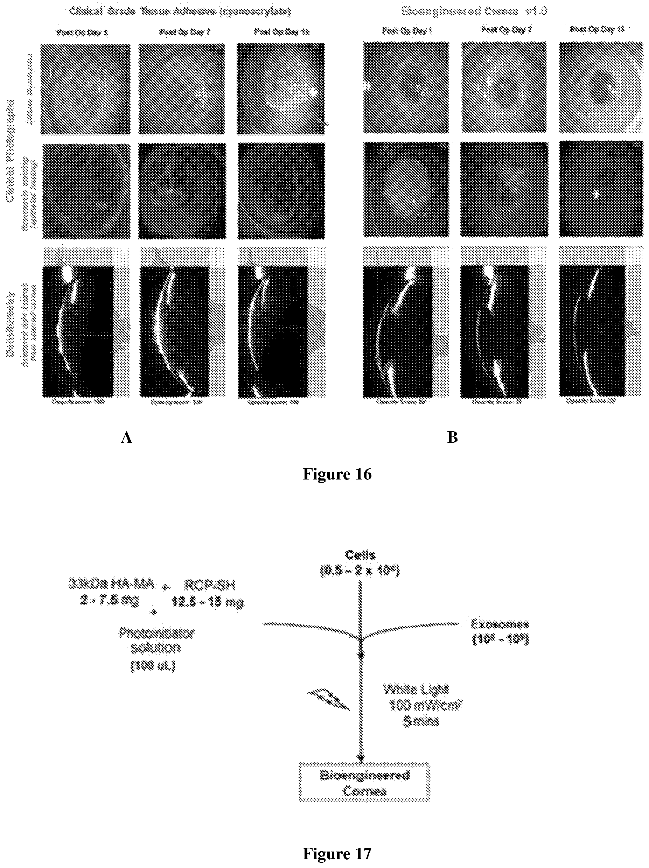

Figure 16 depicts pre-clinical study of hydrogel formulations in rabbit model *in-vivo.* (A) rabbit corneas receiving treatment with cyanoacrylate glue; (B) depicts rabbit corneas receiving hydrogel formulations of the present disclosure, in accordance with an embodiment of the present disclosure.
Figure 17 depicts the optimized protocol for preparing the bioengineered formulation comprising stem cells and exosomes, in accordance with an embodiment of the present disclosure.

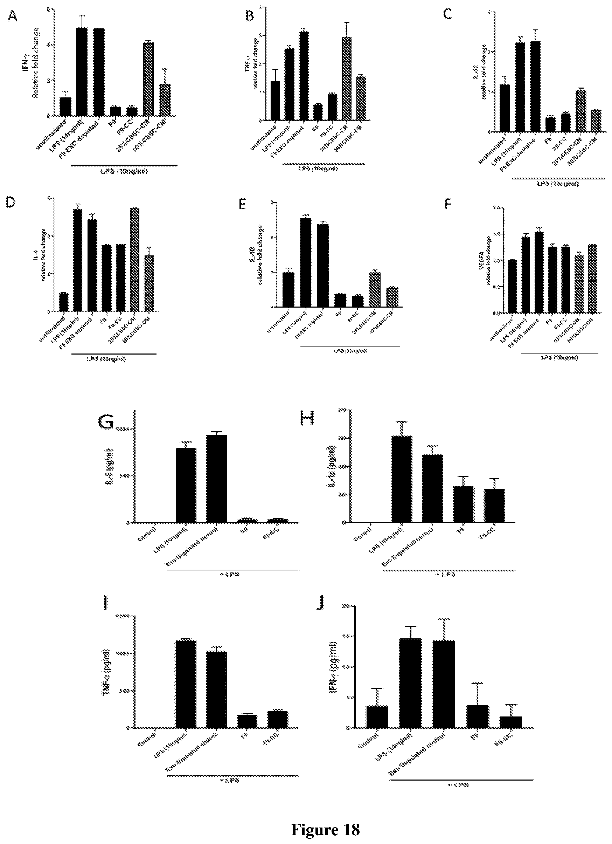

Figure 18 depicts cytokine expressions (transcripts) which were measured for IFNγ **(A),** TNF-α **(B),** IL-1β **(C),** IL-6 **(D),** IL-10 **(E)** and VEGFA **(F),** in accordance with an embodiment of the present disclosure.

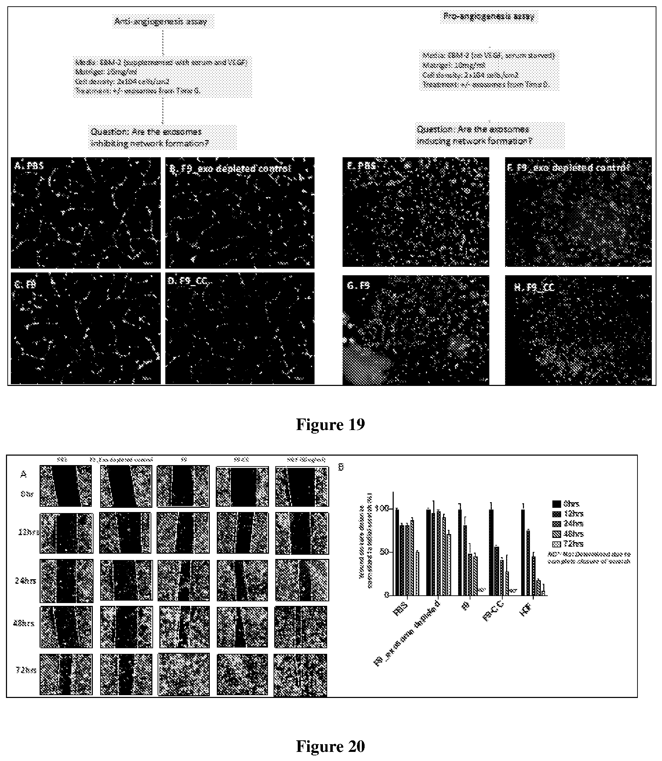

Figure 19 depicts angiogenic activity of hBM-MSC exosomes. In the anti-angiogenesis assay, no significant differences in tube formation were observed between control **(A-B)** and hBM-MSC exosome **(C-D)** treated endothelial cells. In the pro-angiogenesis assay, no significant differences in tube formation were observed between control **(E-F)** and hBM-MSC exosome **(G-H)** treated endothelial cells, in accordance with an embodiment of the present disclosure.
Figure 20 depicts functional characterization of exosomes isolated from hBM-MSCs. **(A)** Representative images depicting the time course of the wound closure (2D scratch assay) on a monolayer of human corneal epithelial cells, observed across multiple time points (0, 12, 24, 48, 72 h); **(B)** Quantification of percentage of wound closure (2D scratch assay) in a monolayer of human corneal epithelial cells at 72 h. Exosomes (4 x 10⁸) from fraction F9 and captocore fraction F9-CC (1 µg) showed potent wound healing capacity, in accordance with an embodiment of the present disclosure.

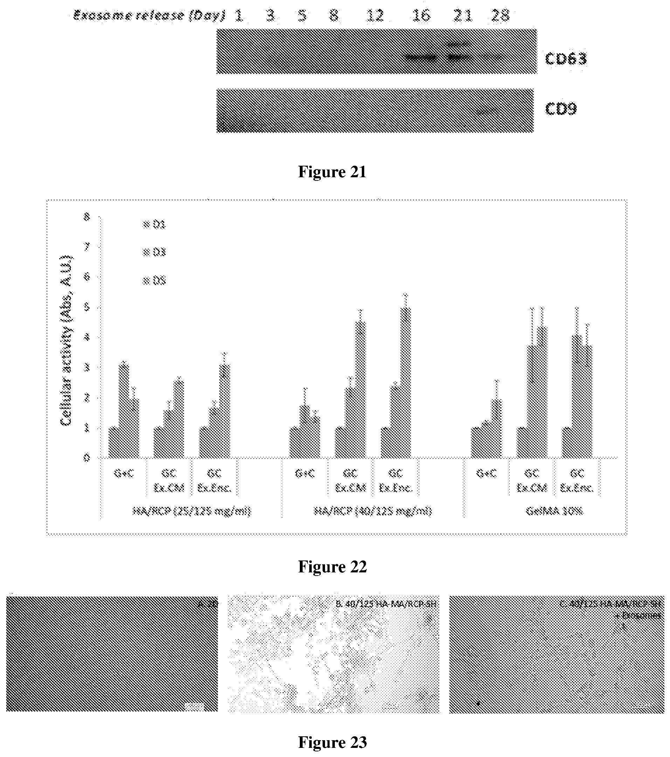

Figure 21 depicts the release of encapsulated exosomes from the hydrogel formulation of the present disclosure. Biopolymer (HA/MA+ RCP-SH) encapsulated exosome release was quantified by western blotting. Hydrogels were mixed with exosomes and incubated in PBS at 37°C for indicated timepoints. Supernatant was collected at different timepoints and analyzed for the expression of exosome specific marker CD63. Sustained release of exosomes was detected by western blot from Day 16 to Day 28. Key: Exosomes in CM: Exosomes in culture media; Encap.: hydrogel encapsulated exosomes, in accordance with an embodiment of the present disclosure.
Figure 22 depicts the influence of exosomes on the cell viability of encapsulated MSCs in the hydrogel formulation of the present disclosure. In the presence of exosomes, cell viability of encapsulated MSCs was considerably higher for HA/RCP (mg/ml, DoS 50%) hydrogels on day 5 compared to cell only acting as a control. G+C: Hydrogel with cells, GC Ex.CM: Cell encapsulated gels receiving exosomes via culture medium, GC Ex.Enc.: Cells and exosomes are encapsulated together in gels, GelMA: Gelatin methacrylate. Data is represented as mean±SE with n=3 replicates, in accordance with an embodiment of the present disclosure.
Figure 23 depicts culture of human dermal fibroblasts in **(A)** 2D; **(B)** on the surface of 40/125 HA-MA/RCP-SH and (C) on the surface of 40/125 HA-MA/RCP-SH with exosome supplemented media. Exosomes did not appear to have any cytotoxic effect on the cells at the concentration used across all studies (0.4 billion exosomes/ml), in accordance with an embodiment of the present disclosure.

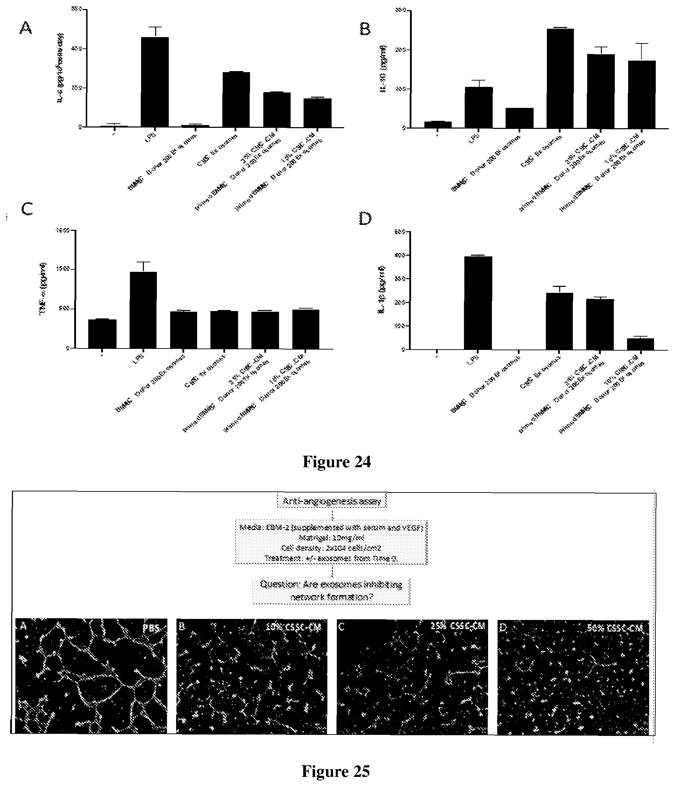

Figure 24 depicts anti-inflammatory effect of CLSC and CLSC-CM primed BMMSC-derived exosomes on RAW 264.7 macrophage cells. RAW 264.7 macrophage cells were treated either with 4x10⁸ exosomes (1µg) followed by LPS stimulation for 4 h. The levels of secreted cytokines were quantified by ELISA for **(A)** IL-6, **(B)** IL-10, **(C)** TNF-α and **(D)** IL-1β, in accordance with an embodiment of the present disclosure.
Figure 25 depicts the angiogenesis activity of CLSC-CM/secretome, in accordance with an embodiment of the present disclosure.

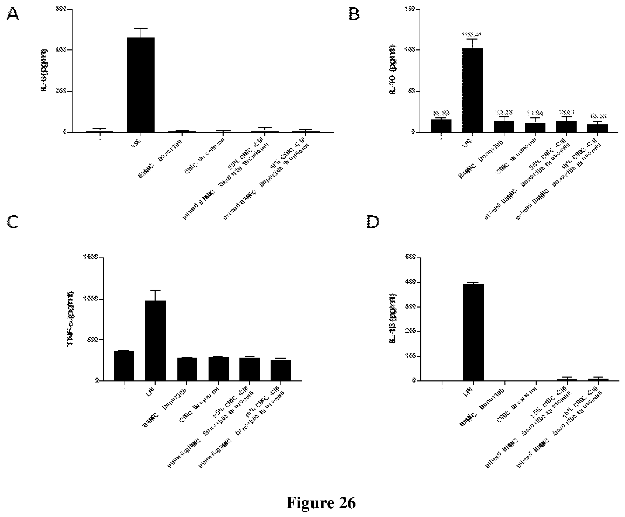

Figure 26 depicts anti-inflammatory effect of CLSC-Conditioned media/secretome and CLSC-CM primed BMMSC conditioned media/secretome on RAW 264.7 macrophage cells. RAW 264.7 macrophage cells were treated either with conditioned media collected from CLSCs and CLSC-CM primed BMMSCs at 50% supplementation (collected from 0.5 million BMMSCs) followed by LPS stimulation for 4 h. The levels of secreted cytokines were also quantified by ELISA for (A) IL-6, **(B)** IL-10, **(C)** TNF-α and **(D)** IL-1β, in accordance with an embodiment of the present disclosure.

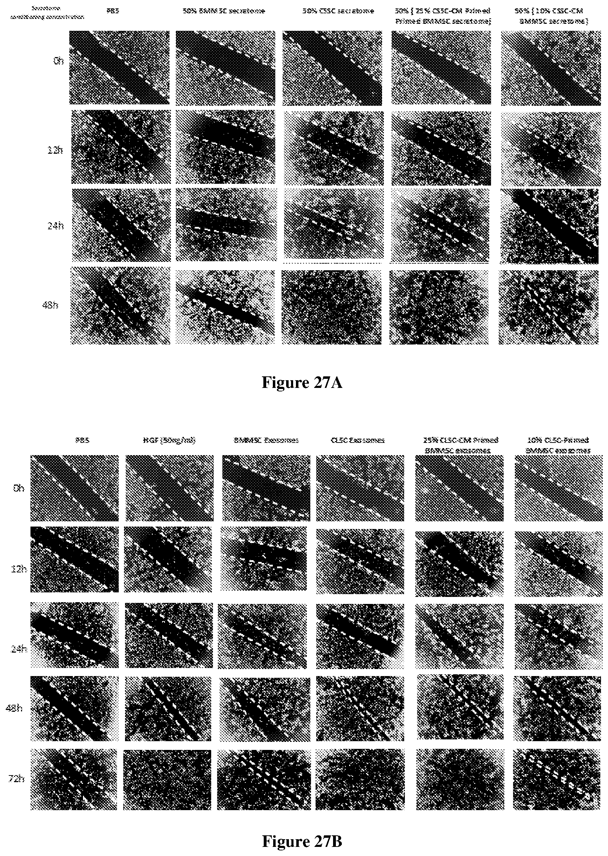

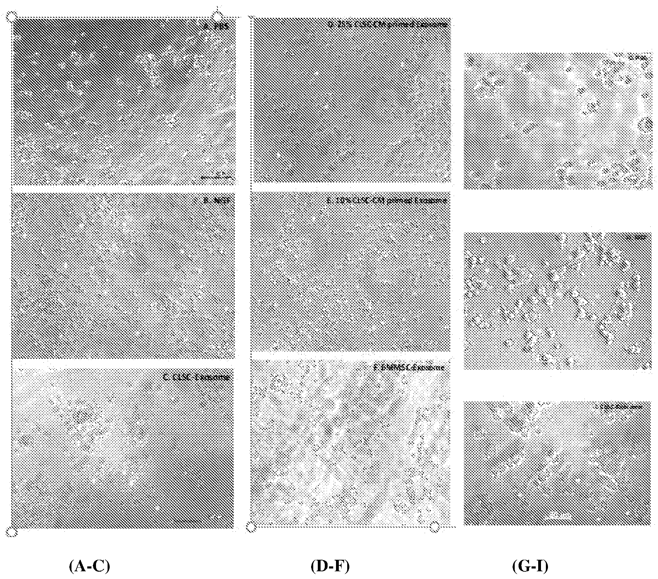

Figure 27 shows that comparison of the wound healing activity of CLSCs, CLSC-CM primed BMMSCs, and BMMSC secretome. **(A)** Representative images depicting the time course of the wound closure (2D scratch assay) on a monolayer of human corneal epithelial cells in the presence of secretome (equivalent to 0.2 million source MSCs), observed across multiple time points (0, 12, 24, 48 h); **(B)** Representative images depicting the time course of the wound closure (2D scratch assay) on a monolayer of human corneal epithelial cells in the presence of 4 x 10⁸ exosomes (equivalent to 0.2 million source MSCs), observed across multiple time points (0, 12, 24, 48, 72 h), in accordance with an embodiment of the present disclosure.

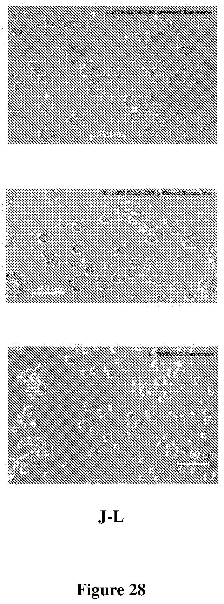

Figure 28 depicts the results of results of the *in-vitro* innervation assay. CLSC-exosomes and CLSC-CM primed BMMSC exosomes promote innervation (neurite outgrowth) in PC12 cells at 0.4 billion exosomes/ml. Cells were treated with 20 ng/ml NGF as a positive control **(B, H). (A-F)** Scale bar: 100 µm; **(G-L)** Scale bar: 50 µm and 100 µm. Yellow arrow indicates neurite outgrowth, in accordance with an embodiment of the present disclosure.

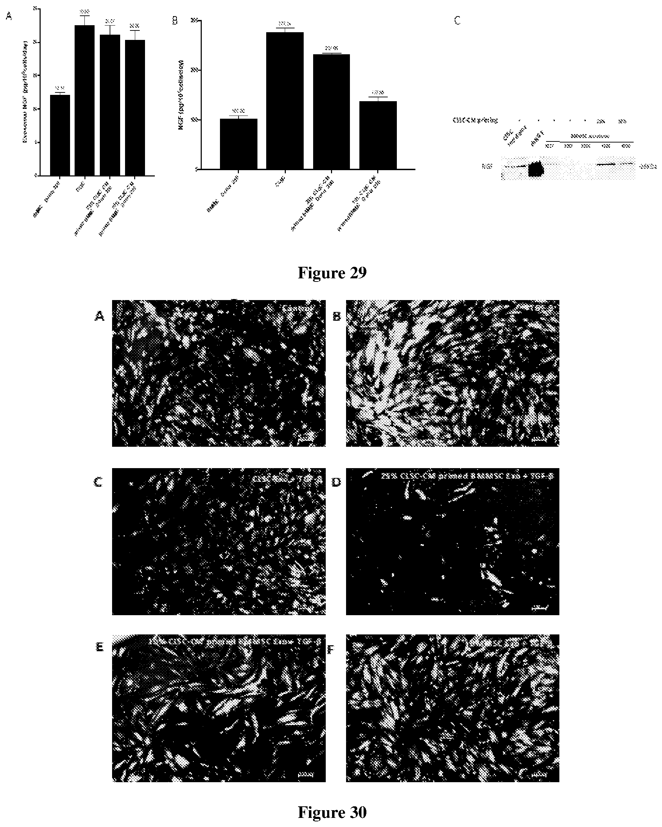

Figure 29 depicts the secreted levels of NGF in **(A)** Exosomes and **(B-C)** Secretome from BMMSCs, CLSCs and CLSC-CM primed BMMSCs, in accordance with an embodiment of the present disclosure.
Figure 30 depicts the anti-fibrotic effect of CLSC-exosomes and CLSC-CM primed exosomes: Human dermal fibroblasts were pre-treated with indicated exosomes (4x10⁸/ml) for 4 hours prior to induction of fibrosis with TGF-β (10ng/ml) for 24 hours. Cells were stained with anti-α-SMA antibody (as indicated with green color) (a marker of fibrosis) and DAPI (nucleus, indicated with blue color), in accordance with an embodiment of the present disclosure.

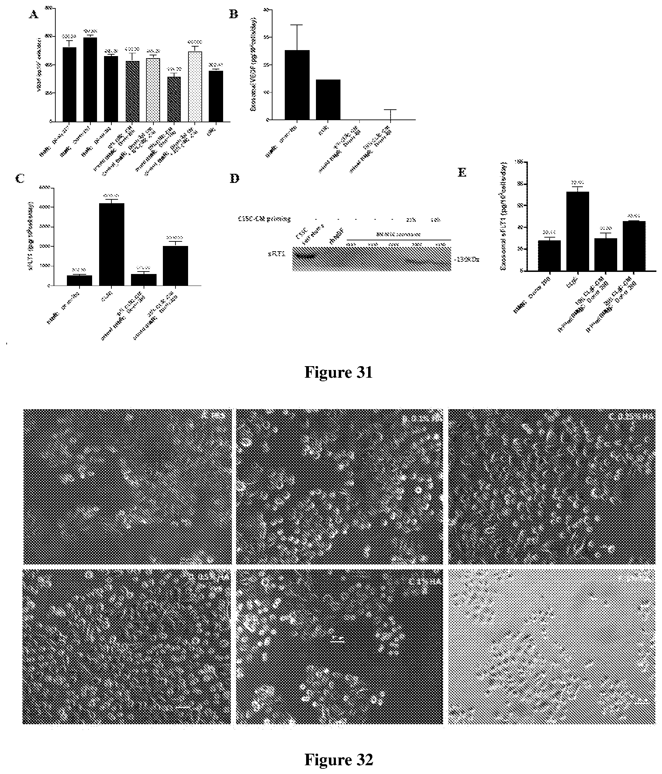

Figure 31 depicts characterisation of angiogenic activity of CLSC and CLSC-CM primed BMMSC-derived exosomes and secretome: VEGF protein levels in **(A)** secretome; **(B)** Exosomes and sFLT1 protein levels in **(C-D)** Secretome; **(E)** Exosomes, in accordance with an embodiment of the present disclosure.
Figure 32 depicts the cellular uptake of CLSC-CM primed BMMSC-derived exosomes in the eyedrop formulation by Human Corneal Epithelial Cells at 4 h. Exosomes were labelled with PKH26 (indicated with red color) and live imaging was undertaken at 4hours. Uptake of exosomes was observed across all tested formulations, in accordance with an embodiment of the present disclosure.

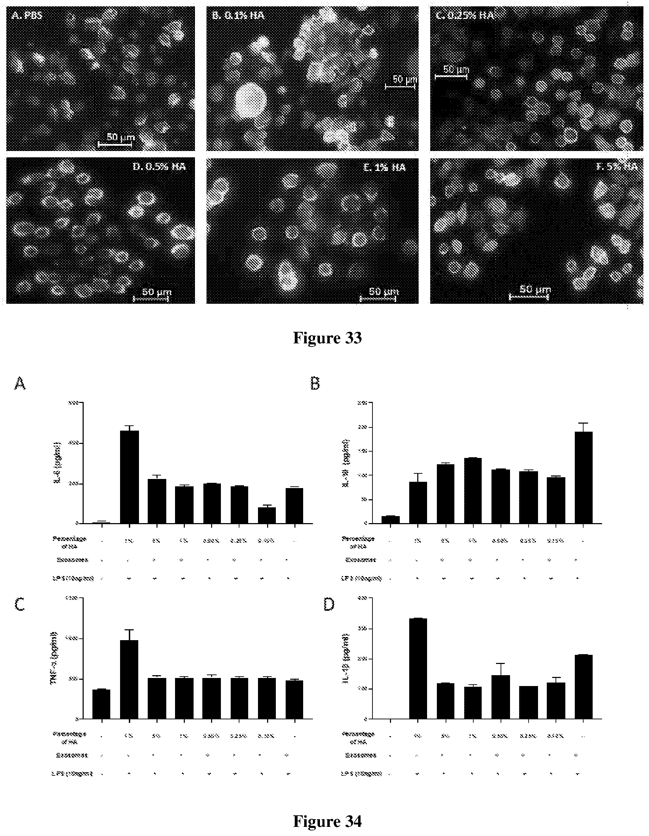

Figure 33 depicts the cellular uptake of CLSC-CM primed BMMSC - derived exosomes in the eye drop formulation by Human Corneal Epithelial Cells at 4 h. Exosomes were labelled with PKH26 (indicated with red color) and incubated with cells for 4 hours. Cells were fixed and labelled with Cytokeratin-3 (indicated with green color) and DAPI (indicated with blue color), in accordance with an embodiment of the present disclosure.
Figure 34 depicts the anti-inflammatory activity of eyedrop formulation. RAW 264.7 cells were activated with LPS in the presence or absence of exosomes (25% CLSC-CM primed BMMSC-exosomes) (4x10⁸ exosomes/ml) reconstituted in the above indicated concentrations of clinical grade HA (0.1-5%). As seen above, varied concentrations of HA did not impact the anti-inflammatory activity of CLSC-CM primed exosomes, ensuring the therapeutic effect of our exosomes in an eyedrop formulation format. The secretion of (A) IL-6; (B) IL-10; (C) TNF-α and (D) IL-1β were quantified by ELISA as previously described, in accordance with an embodiment of the present disclosure.

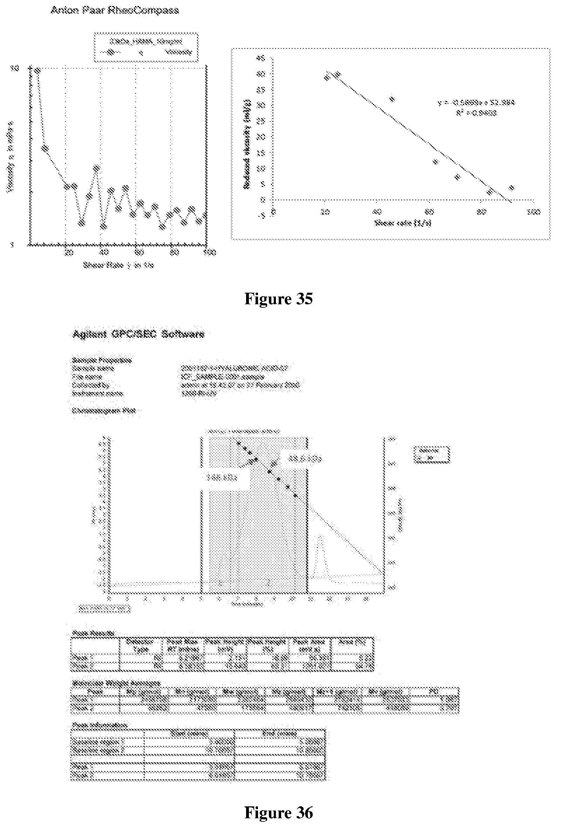

Figure 35 depicts representative raw data obtained from rheometer for the calculation of intrinsic viscosity. Intrinsic viscosity is defined as the viscosity at shear rate approaching 0, in accordance with an embodiment of the present disclosure.
Figure 36 depicts GPC data of "33 kDa" HA raw material obtained from Stanford Chemicals, in accordance with an embodiment of the present disclosure.

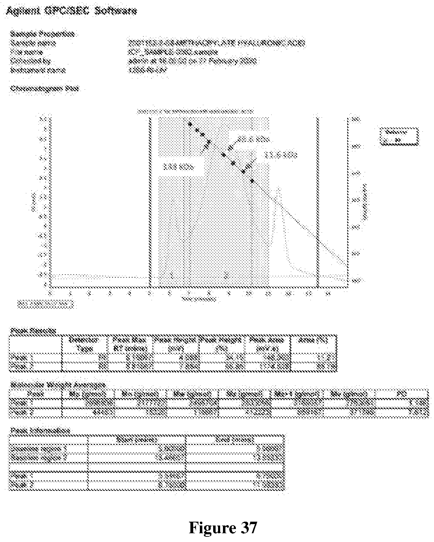

Figure 37 depicts GPC data of "33 kDa" HA methacrylate derived from raw material (Stanford Chemicals) and derivatized by CreativePEG Works, in accordance with an embodiment of the present disclosure.

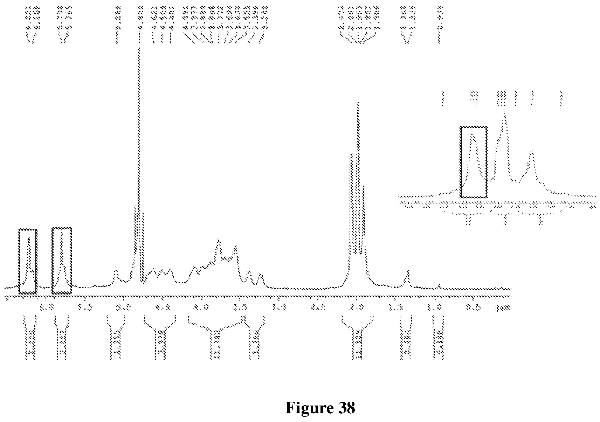

Figure 38 depicts the representative H-NMR data of "33 kDa" HA-MA, in accordance with an embodiment of the present disclosure.

### DETAILED DESCRIPTION OF THE INVENTION

Those skilled in the art will be aware that the present disclosure is subject to variations and modifications other than those specifically described. It is to be understood that the present disclosure includes all such variations and modifications. The disclosure also includes all such steps, features, compositions, and compounds referred to or indicated in this specification, individually or collectively, and any and all combinations of any or more of such steps or features.

### Definitions

For convenience, before further description of the present disclosure, certain terms employed in the specification, and examples are delineated here. These definitions should be read in the light of the remainder of the disclosure and understood as by a person of skill in the art. The terms used herein have the meanings recognized and known to those of skill in the art, however, for convenience and completeness, particular terms and their meanings are set forth below.

The articles "a", "an" and "the" are used to refer to one or to more than one (i.e., to at least one) of the grammatical object of the article.

The terms "comprise" and "comprising" are used in the inclusive, open sense, meaning that additional elements may be included. It is not intended to be construed as "consists of only".

Throughout this specification, unless the context requires otherwise the word "comprise", and variations such as "comprises" and "comprising", will be understood to imply the inclusion of a stated element or step or group of element or steps but not the exclusion of any other element or step or group of element or steps.

The term "including" is used to mean "including but not limited to". "Including" and "including but not limited to" are used interchangeably.

For the purposes of the present document, the term "bioengineered formulation" refers to a polymer mixture of different compositions. In the present disclosure, the terms "bioengineered formulation" and "hydrogel formulation" are used interchangeably. The cross-linking process starts after the addition of photo-initiator, however, the cross-linking gets completed only after the exposure of white light of certain intensity as disclosed in the present disclosure. As a person skilled in the art would understand that testing of certain parameters like molecular weight, degree of substitution, compressive modulus and tensile strength would only be possible in the cross-linked product like hydrogel. Molecular weight and degree or substitution are the property of the biopolymers, that differentiate them from other polymers comprising of same chain.

The degree of substitution (DOS) of a polymer is the (average) number of substituent groups attached per base unit (in the case of condensation polymers) or per monomeric unit (in the case of addition polymers).

The terms "collagen" and "collagen sequence derived peptide" as used herein is used to include natural, synthetic, recombinant and/or alternate versions of said polypeptide and protein sequences.

The term "modified hyaluronic acid" or "modified collagen peptide" or "modified collagen", or "modified silk" or "modified cellulose" or "polyethylene glycol" or "modified polyvinyl alcohol" or "modified alginate" denotes any kind of modification that is possible in the respective molecules. The specific modifications that have been done are covered in the presented disclosure. For example, modified cellulose intends to mean the modified molecules like methyl cellulose, carboxymethyl cellulose (CMC), hydroxypropyl methyl cellulose (HPMC) and hydroxyethyl methyl cellulose (HEMC).

The term "mesenchymal stem cell derived-conditioned medium or "MSC-CM" refers to the medium obtained after the growth of the MSC. The conditioned medium thus obtained comprises secreted cell modulators and multiple factors critical for tissue regeneration. The conditioned medium thus obtained also comprises secretome, and exosomes which needs to be purified from the conditioned medium before being able to apply for therapeutic purposes. The process for obtaining expanded MSC as described herein also leads to the formation of MSC-CM, therefore, it can be said that a single process leads to the procurement of a population of expanded MSC as well as of MSC-CM. The term "exosomes" refers to the type of an extracellular vesicle that contain constituents (in terms of protein, DNA, and RNA) of the biological cells that secretes them. The exosomes obtained from the conditioned medium as described herein is used for therapeutic purposes.

The term "corneal stromal stem cell derived-conditioned medium or "CSSC-CM" refers to the medium in which corneal stromal stem cells (CSSC) are grown. The CSSC-CM as described herein is obtained by culturing of CSSC in a manner known in the art or by culturing of CSSC as per the method disclosed herein. Corneal Limbal Stem Cells (CLSC) are isolated from the limbal ring as described in previous PCT Applications; PCT/IN2020/050622 & PCT/IN2020/050623. These cells can be divided into two subpopulations: corneal stromal stem cells (CSSC) and Limbal Epithelial Stem Cells (LESC). The PCT Application PCT/IN2020/050622 & PCT/IN2020050623 disclose methods for CSSC isolation and demonstrates enrichment of CSSC population over LESCs by the protocol used therein. However, in case there is a small population of LESCs left behind in the CSSC enriched fraction, the same is being referred to as 'CLSC' to cover all cell types in these applications. Therefore, the conditioned medium derived from such CSSC enriched population is known as CSSC-derived conditioned medium (CSSC-CM). It is understood that for the sake of simplicity, the term CSSC-CM is also used to denote the conditioned medium obtained by culturing enriched CSSC in which a small population of LESC is also present.

The term "xeno-free" as described in the present disclosure refers to the process as described herein which is free of any product which is derived from non-human animal. The method being xeno-free is an important advantage because of its plausibility of clinical application. The term "scalable" refers to the ability to increase the production output manifolds. The term "subject" refers to a human subject who is suffering from the conditions as mentioned in the present disclosure. The term "therapeutically effective amount" refers to the amount of a composition which is required for treating the conditions of a subject.

The term "culture medium" refers to the medium in which the MSC is cultured. The culture medium comprises MSC basal medium, and the MSC basal medium is used as per the MSC which is being cultured. The MSC basal medium as mentioned in the present disclosure was commercially procured. For the purposes of the present disclosure, RoosterBio xenofree media was used for BMMSCs.

The term "conditioned medium" refers to the media enriched with cell secreted factors such as various proteins/growth factors, such as hepatocyte growth factor (HGF), keratocyte growth factor (KGF) and soluble form like tyrosine kinase1 (sFLT1), Pigment epithelial-derived growth factor (PEDF) ,thrombospondin and exosomes containing various molecules including miR-10b, miR-21, miR-23a, miR-182, miR-181a, miR-145 and epidermal growth factor (EGF), fibroblast growth factor (FGF), sFLT1 and phosphoglycerate kinase (PGK), phosphoglucomutase, enolase, CD73, CD63 and MMP9. The composition of conditioned medium is intended to be exploited for therapeutic applications. The term "cell modulators" refers to various secreted factors such as ECM, growth factors, exosomal cargos containing a broad range of small and macromolecules, many of protein or nucleic acid in nature. Some of these include micro-RNA, mRNA, long non-coding RNA, lipid mediator, that can modulate cellular response. The term "exosomes" refers to cell secreted vesicles containing cargo molecules of protein or nucleic acid in nature, often referring to the 20-200 nm range with molecules of clinical interest such as, anti-inflammatory, anti-fibrotic and regenerative properties.

The term "corneal defect" or "corneal disorder" have been used interchangeably to denote the issues in the cornea which require medical intervention. The intervention can be to an extent of replacing the damaged corneal with the bio-printed lenticule as described in the present disclosure.

Ratios, concentrations, amounts, and other numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited.

To overcome the problems faced in the art, the present disclosure provides a bioengineered formulation comprising the combination of the polymers that facilitates proper cross-linking of bioengineered formulation, and which can be used for non-invasive, quick and long-term repair of corneal stromal defects.

The present disclosure provides a bioengineered formulation comprising a combination of a modified collagen peptide and a modified hyaluronic acid. The use of the combination of the modified collagen peptide having molecular weight in the range of 20-80 kDa, and with a degree of substitution in the range of 20-75%, and the modified hyaluronic acid having a molecular weight in the range of 10-100 kDa, and with a degree of substitution in the range of 20-75% helps in improving the physical and biomechanical characteristics of the bioengineered formulation. The bioengineered formulation of the present disclosure is cross-linked with a photoinitiator in the presence of light to yield a transparent crosslinked hydrogel that firmly adheres to the corneal tissue. Further, the bioengineered formulation is biomimetic as it possesses the physical, mechanical and biological properties that match the characteristics of native cornea tissue. For instance, the bioengineered formulation of the present disclosure has a compressive modulus in the range of 100-1400 kPa, preferably 100-500 kPa. Moreover, the bioengineered formulation is resistant to at most 50% degradation within 28 days under *in-vitro* conditions. Moreover, the bioengineered formulation of the present disclosure promotes human corneal epithelial cell migration and proliferation supporting surface epithelialization and thereby, confirming biocompatibility and cornea-mimetic properties.

The bioengineered formulation of the present disclosure further comprises stem cells, or exosomes, or combinations thereof, encapsulated in the bioengineered formulation exhibits anti-fibrotic, anti-angiogenic, anti-inflammatory and pro-reinnervation properties. The addition of exosomes in the bioengineered formulation helps in addressing a range of corneal injuries and dystrophies due to the highly therapeutic advantages of exosomes, which includes low immunogenicity and tumorigenicity, tissue specific homing capability and low risk of embolism formation. The bioengineered formulation of the present disclosure also promotes the sustained release of stem cells, or exosomes, or combinations thereof at the site of corneal defect for a longer period of time and helps in enhancing the wound healing capacity of the formulation. The present disclosure also provides a method of treating corneal defect or corneal disorder comprising the step of applying the suitable amount of bioengineered formulation at the site of corneal defect, and illuminating a white light having an intensity in the range of 50-150mW/cm² on the formulation at the site of the corneal defect for a time period in a range of 1-15 minutes, preferably, 2-8 minutes, for treating the corneal defect in a subject. The application of the highly transparent bioengineered formulation at the site of the corneal defect helps in promoting scar-less wound healing of cornea. The bioengineered formulation of the present disclosure helps in treating corneal defect or corneal diseases, including but not limited to anterior corneal scarring involving epithelial and stromal injuries/infection (active inflammation), Stage 1 neurotrophic keratitis (NK) (persistent corneal epithelial defect), Stage 2 NK (large persistent epithelial defect characterized by smooth, rolled edges), Stage 3 NK (deep corneal ulcer, stromal melting, and sterile hypopyon), corneal ulcers such as Mooren's ulcer, keratoconus and corneal perforations. The bioengineered formulation of the present disclosure also helps in treating corneal limbal injuries and corneal dystrophies (CDs), such as lattice CD type 1, granular CD type 1, and congenital stromal CD, wherein the corneal stroma is damaged in the subject. Moreover, the bioengineered formulation of the present disclosure acts as potential treatment for Schnyder CD and lattice CD type-2, wherein both the epithelium and stroma are compromised.

The use of the bioengineered formulation of the present disclosure is followed by post-operative care using exosomal eye drops (post hydrogel application) that allow sustained release of stem cells, or exosomes, or combinations thereof over a period of time, which not only enhances efficient re-epithelialization but also promotes resolution of injury-induced fibrosis and inflammation surrounding the injury. Moreover, the present disclosure provides a formulation comprising: (a) exosomes selected from the group consisting of corneal stromal stem cell derived-exosomes, primed mesenchymal stem cell derived-exosomes, and naive mesenchymal stem cell derived-exosomes; and (b) a clinically approved eye drop formulation. The combination of encapsulated exosomes with clinically approved eye drop formulation allows suppression of any inflammatory responses and gradual healing of fibrotic scars with no neovascularization.

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the disclosure, the preferred methods, and materials are now described. All publications mentioned herein are incorporated herein by reference.

The present disclosure is not to be limited in scope by the specific embodiments described herein, which are intended for the purposes of exemplification only. Functionally-equivalent products, compositions, and methods are clearly within the scope of the disclosure, as described herein.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%. In another embodiment of the present disclosure, the modified collagen peptide having a molecular weight in the range of 25-75 kDa, and with a degree of substitution in the range of 25-70%. In yet another embodiment of the present disclosure, the modified collagen peptide having a molecular weight in the range of 30-70 kDa, and with a degree of substitution in the range of 35-60%. In one another embodiment of the present disclosure, the modified collagen peptide having a molecular weight in the range of 40-60 kDa, and with a degree of substitution in the range of 40-55%.

**In** an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 12-48 kDa, and with a degree of substitution in the range of 20-75%. In another embodiment of the present disclosure, the modified hyaluronic acid having a molecular weight in the range of 15-45 kDa, and with a degree of substitution in the range of 25-70%. In yet another embodiment of the present disclosure, the modified hyaluronic acid having a molecular weight in the range of 20-40 kDa, and with a degree of substitution in the range of 35-65%. In one another embodiment of present disclosure, the modified hyaluronic acid having a molecular weight in the range of 25-35 kDa, and with a degree of substitution in the range of 50-55%.

**In** an embodiment of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa. In yet another embodiment of the present disclosure, the first polymer is having a molecular weight in a range of 30-70 kDa, and the second polymer is having a molecular weight in a range of 30-37 kDa.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%, wherein the bioengineered formulation is cross-linked.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%, and wherein the modified hyaluronic acid having a molecular weight in the range of 12-48 kDa.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%, wherein the modified collagen peptide is in the concentration range of 20-250 mg/ml with respect to the bioengineered formulation, and wherein the modified hyaluronic acid is in the concentration range of 20-80 mg/ml with respect to the bioengineered formulation. In another embodiment of the present disclosure, the modified collagen peptide is in the concentration range of 30-220 mg/ml with respect to the bioengineered formulation, and wherein the modified hyaluronic acid is in the concentration range of 25-75 mg/ml with respect to the bioengineered formulation. In yet another embodiment of the present disclosure, the modified collagen peptide is in the concentration range of 40-200 mg/ml with respect to the bioengineered formulation, and wherein the modified hyaluronic acid is in the concentration range of 30-60 mg/ml with respect to the bioengineered formulation. In one another embodiment of the present disclosure, the modified collagen peptide is in the concentration range of 50-175 mg/ml with respect to the bioengineered formulation, and wherein the modified hyaluronic acid is in the concentration range of 32-50 mg/ml with respect to the bioengineered formulation.

In an embodiment of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa, and wherein the modified hyaluronic acid having a molecular weight in the range of 12-48 kDa, and wherein the modified collagen peptide is in the concentration range of 20-250 mg/ml with respect to the bioengineered formulation, and wherein the modified hyaluronic acid is in the concentration range of 20-80 mg/ml with respect to the bioengineered formulation.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%, wherein the modified collagen peptide is selected from the group consisting of thiolated collagen peptide, and methacrylated collagen peptide. In another embodiment of the present disclosure, the modified collagen peptide is thiolated collagen peptide. In yet another embodiment of the present disclosure, the modified collagen peptide is methacrylated collagen peptide.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%, wherein the modified hyaluronic acid is selected from the group consisting of methacrylated hyaluronic acid, and thiolated hyaluronic acid. In another embodiment of the present disclosure, the modified hyaluronic acid is methacrylated hyaluronic acid. In yet another embodiment of the present disclosure, the modified hyaluronic acid is thiolated hyaluronic acid.

In an embodiment of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa, and wherein the modified collagen peptide is selected from the group consisting of a thiolated collagen peptide, and a methacrylated collagen peptide.

In an embodiment of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa, and wherein the modified hyaluronic acid is selected from the group consisting of a methacrylated hyaluronic acid, and a thiolated hyaluronic acid.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%, wherein the modified collagen peptide is in the concentration range of 20-250 mg/ml with respect to the bioengineered formulation, and wherein the modified hyaluronic acid is in the concentration range of 20-80 mg/ml with respect to the bioengineered formulation, and wherein the modified collagen peptide is selected from the group consisting of thiolated collagen peptide, and methacrylated collagen peptide, and wherein the modified hyaluronic acid is selected from the group consisting of methacrylated hyaluronic acid, and thiolated hyaluronic acid.

In an embodiment of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa, and wherein the modified collagen peptide is in the concentration range of 20-250 mg/ml with respect to the bioengineered formulation, and wherein the modified hyaluronic acid is in the concentration range of 20-80 mg/ml with respect to the bioengineered formulation, and wherein the modified collagen peptide is selected from the group consisting of thiolated collagen peptide, and methacrylated collagen peptide, ,and wherein the modified hyaluronic acid is selected from the group consisting of methacrylated hyaluronic acid, and thiolated hyaluronic acid.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%; and (c) at least one type of stem cells selected from the group consisting of mesenchymal stem cells, corneal stromal stem cells, corneal limbal stem cells, and induced pluripotent stem cells. In another embodiment of the present disclosure, the at least one type of stem cells is mesenchymal stem cells. In yet another embodiment of the present disclosure, the at least one type of stem cells is corneal stromal stem cells. In one another embodiment of the present disclosure, the at least one type of stem cells is corneal limbal stem cells.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%; and (c) at least one type of stem cells selected from the group consisting of mesenchymal stem cells, corneal stromal stem cells, corneal limbal stem cells, and induced pluripotent stem cells, wherein the mesenchymal stem cell is selected from the group consisting of human bone marrow- mesenchymal stem cell, adipose tissue- mesenchymal stem cell, umbilical cord- mesenchymal stem cell, Wharton jelly- mesenchymal stem cell, dental pulp-derived mesenchymal stem cell, and corneal limbal stem cell-derived conditioned media primed mesenchymal stem cells.

In an embodiment of the present disclosure, there is provided a bioengineered formulation comprising: (a) a first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) at least one type of stem cells selected from the group consisting of mesenchymal stem cells, corneal stromal stem cells, corneal limbal stem cells, and induced pluripotent stem cells.

In an embodiment of the present disclosure, there is provided a bioengineered formulation comprising: (aa first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) at least one type of stem cells selected from the group consisting of mesenchymal stem cells, corneal stromal stem cells, corneal limbal stem cells, and induced pluripotent stem cells, wherein the mesenchymal stem cell is selected from the group consisting of human bone marrow- mesenchymal stem cell, adipose tissue- mesenchymal stem cell, umbilical cord- mesenchymal stem cell, Wharton jelly- mesenchymal stem cell, dental pulp-derived mesenchymal stem cell, and corneal limbal stem cell-derived conditioned media primed mesenchymal stem cells.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%; and (c) at least one type of stem cells selected from the group consisting of mesenchymal stem cells, corneal stromal stem cells, corneal limbal stem cells, and induced pluripotent stem cells, wherein the stem cells are present in the range of 0.1-10 million cells. In another embodiment of the present disclosure, the stem cells are present in the range of 0.4-9 million cells, or 0.5-7 million cells, or 1-5 million cells.

In an embodiment of the present disclosure, there is provided a bioengineered formulation comprising: (a) a first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) at least one type of stem cells selected from the group consisting of mesenchymal stem cells, corneal stromal stem cells, corneal limbal stem cells, and induced pluripotent stem cells, wherein the stem cells are present in the range of 0.1-10 million cells.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%; and (c) at least one type of stem cells selected from the group consisting of mesenchymal stem cells, corneal stromal stem cells, corneal limbal stem cells, and induced pluripotent stem cells, wherein the mesenchymal stem cell is selected from the group consisting of human bone marrow- mesenchymal stem cell, adipose tissue- mesenchymal stem cell, umbilical cord- mesenchymal stem cell, Wharton jelly- mesenchymal stem cell, dental pulp-derived mesenchymal stem cell, and corneal limbal stem cell-derived conditioned media primed mesenchymal stem cells, and wherein the stem cells are present in the range of 0.1-10 million cells.

In an embodiment of the present disclosure, there is provided a bioengineered formulation comprising: (a) a first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) at least one type of stem cells selected from the group consisting of mesenchymal stem cells, corneal stromal stem cells, corneal limbal stem cells, and induced pluripotent stem cells, wherein the mesenchymal stem cell is selected from the group consisting of human bone marrow- mesenchymal stem cell, adipose tissue- mesenchymal stem cell, umbilical cord- mesenchymal stem cell, Wharton jelly- mesenchymal stem cell, dental pulp-derived mesenchymal stem cell, and corneal limbal stem cell-derived conditioned media primed mesenchymal stem cells, and wherein the stem cells are present in the range of 0.1-10 million cells.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%; and (c) exosomes selected from the group consisting of corneal stromal stem cell derived-exosomes, primed mesenchymal stem cell derived-exosomes, and naive mesenchymal stem cell derived-exosomes.

In an embodiment of the present disclosure, there is provided a bioengineered formulation comprising: (a) a first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) exosomes selected from the group consisting of corneal stromal stem cell derived-exosomes, primed mesenchymal stem cell derived-exosomes, and naive mesenchymal stem cell derived-exosomes.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%; and (c) exosomes selected from the group consisting of corneal stromal stem cell derived-exosomes, primed mesenchymal stem cell derived-exosomes, and naive mesenchymal stem cell derived-exosomes, wherein the exosomes has a concentration in the range of 0.5-25 billion exosomes per ml of the bioengineered formulation. In another embodiment of the present disclosure, the exosomes has a concentration in the range of 1.0-20 billion exosomes per ml of the bioengineered formulation. In yet another embodiment of the present disclosure, the exosomes has a concentration in the range of 5.0-15 billion exosomes per ml of the bioengineered formulation. In one another embodiment of the present disclosure, the exosomes has a concentration in the range of 7.0-10 billion exosomes per ml of the bioengineered formulation.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%; and (c) exosomes selected from the group consisting of corneal stromal stem cell derived-exosomes, primed mesenchymal stem cell derived-exosomes, and naive mesenchymal stem cell derived-exosomes, wherein the primed mesenchymal stem cell derived-exosomes are exosomes derived from mesenchymal stem cells primed with corneal stromal stem cell derived-conditioned medium.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%; and (c) exosomes selected from the group consisting of corneal stromal stem cell derived-exosomes, primed mesenchymal stem cell derived-exosomes, and naive mesenchymal stem cell derived-exosomes, wherein the exosomes has a concentration in the range of 0.5-25 billion exosomes per ml of the bioengineered formulation, and wherein the primed mesenchymal stem cell derived-exosomes are exosomes derived from mesenchymal stem cells primed with corneal stromal stem cell derived-conditioned medium.

In an embodiment of the present disclosure, there is provided a bioengineered formulation comprising: (a) a first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) exosomes selected from the group consisting of corneal stromal stem cell derived-exosomes, primed mesenchymal stem cell derived-exosomes, and naive mesenchymal stem cell derived-exosomes, wherein the exosomes has a concentration in the range of 0.5-25 billion exosomes per ml of the bioengineered formulation.

In an embodiment of the present disclosure, there is provided a bioengineered formulation comprising: (a) a first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) exosomes selected from the group consisting of corneal stromal stem cell derived-exosomes, primed mesenchymal stem cell derived-exosomes, and naive mesenchymal stem cell derived-exosomes, wherein the primed mesenchymal stem cell derived-exosomes are exosomes derived from mesenchymal stem cells primed with corneal stromal stem cell derived-conditioned medium.

In an embodiment of the present disclosure, there is provided a bioengineered formulation comprising: (a) a first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) exosomes selected from the group consisting of corneal stromal stem cell derived-exosomes, primed mesenchymal stem cell derived-exosomes, and naive mesenchymal stem cell derived-exosomes, wherein the exosomes has a concentration in the range of 0.5-25 billion exosomes per ml of the bioengineered formulation, and wherein the primed mesenchymal stem cell derived-exosomes are exosomes derived from mesenchymal stem cells primed with corneal stromal stem cell derived-conditioned medium.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 12-48 kDa, and with a degree of substitution in the range of 20-75%, wherein the modified hyaluronic acid is methacrylated hyaluronic acid, and wherein the modified collagen is thiolated collagen peptide.

In an embodiment of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa, and wherein the modified hyaluronic acid is methacrylated hyaluronic acid, and wherein the modified collagen is thiolated collagen peptide.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%; (c) stem cells selected from the group consisting of mesenchymal stem cells, corneal stromal stem cells, and corneal limbal stem cells; and (d) exosomes selected from the group consisting of corneal stromal stem cell derived-exosomes, primed mesenchymal stem cell derived-exosomes, and naive mesenchymal stem cell derived-exosomes.

In an embodiment of the present disclosure, there is provided a bioengineered formulation comprising: (a) a first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) stem cells selected from the group consisting of mesenchymal stem cells, corneal stromal stem cells, and corneal limbal stem cells; and (d) exosomes selected from the group consisting of corneal stromal stem cell derived-exosomes, primed mesenchymal stem cell derived-exosomes, and naive mesenchymal stem cell derived-exosomes.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%, wherein the modified collagen peptide is in the concentration range of 20-250 mg/ml with respect to the bioengineered formulation, and (c) at least one type of stem cells selected from the group consisting of mesenchymal stem cells, corneal stromal stem cells, corneal limbal stem cells, and induced pluripotent stem cells, wherein the modified hyaluronic acid is in the concentration range of 20-80 mg/ml with respect to the bioengineered formulation, and wherein the modified collagen peptide is selected from the group consisting of thiolated collagen peptide, and methacrylated collagen peptide, , and wherein the modified hyaluronic acid is selected from the group consisting of methacrylated hyaluronic acid, and thiolated hyaluronic acid, and wherein the mesenchymal stem cell is selected from the group consisting of human bone marrow- mesenchymal stem cell, adipose tissue- mesenchymal stem cell, umbilical cord- mesenchymal stem cell, Wharton jelly- mesenchymal stem cell, dental pulp-derived mesenchymal stem cell, and corneal limbal stem cell-derived conditioned media primed mesenchymal stem cells, and wherein the stem cells are present in the range of 0.1-10 million cells.

In an embodiment of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa, and wherein the modified collagen peptide is in the concentration range of 20-250 mg/ml with respect to the bioengineered formulation, and wherein the modified hyaluronic acid is in the concentration range of 20-80 mg/ml with respect to the bioengineered formulation, and wherein the modified collagen peptide is selected from the group consisting of thiolated collagen peptide, and methacrylated collagen peptide, ,and wherein the modified hyaluronic acid is selected from the group consisting of methacrylated hyaluronic acid, and thiolated hyaluronic acid, and (c) at least one type of stem cells selected from the group consisting of mesenchymal stem cells, corneal stromal stem cells, corneal limbal stem cells, and induced pluripotent stem cells, wherein the modified hyaluronic acid is in the concentration range of 20-80 mg/ml with respect to the bioengineered formulation, and wherein the modified collagen peptide is selected from the group consisting of thiolated collagen peptide, and methacrylated collagen peptide, and wherein the modified hyaluronic acid is selected from the group consisting of methacrylated hyaluronic acid, thiolated hyaluronic acid, and wherein the mesenchymal stem cell is selected from the group consisting of human bone marrow- mesenchymal stem cell, adipose tissue- mesenchymal stem cell, umbilical cord- mesenchymal stem cell, Wharton jelly- mesenchymal stem cell, dental pulp-derived mesenchymal stem cell, and corneal limbal stem cell-derived conditioned media primed mesenchymal stem cells, and wherein the stem cells are present in the range of 0.1-10 million cells.

In an embodiment of the present disclosure, there is provided a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 12-48 kDa, and with a degree of substitution in the range of 20-75%, wherein the modified collagen peptide is in the concentration range of 20-250 mg/ml with respect to the bioengineered formulation, and (c) exosomes selected from the group consisting of corneal stromal stem cell derived-exosomes, primed mesenchymal stem cell derived-exosomes, and naive mesenchymal stem cell derived-exosomes, wherein the modified hyaluronic acid is in the concentration range of 20-80 mg/ml with respect to the bioengineered formulation, and wherein the modified collagen peptide is selected from the group consisting of thiolated collagen peptide, and methacrylated collagen peptide, and wherein the modified hyaluronic acid is selected from the group consisting of methacrylated hyaluronic acid, and thiolated hyaluronic acid, and wherein the exosomes has a concentration in the range of 0.5-25 billion exosomes per ml of the bioengineered formulation, and wherein the primed mesenchymal stem cell derived-exosomes are exosomes derived from mesenchymal stem cells primed with corneal stromal stem cell derived-conditioned medium.

In an embodiment of the present disclosure, there is provided a bioengineered formulation comprising: (a) a first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) exosomes selected from the group consisting of corneal stromal stem cell derived-exosomes, primed mesenchymal stem cell derived-exosomes, and naive mesenchymal stem cell derived-exosomes, wherein the modified hyaluronic acid is in the concentration range of 20-80 mg/ml with respect to the bioengineered formulation, and wherein the modified collagen peptide is selected from the group consisting of thiolated collagen peptide, and methacrylated collagen peptide, and wherein the modified hyaluronic acid is selected from the group consisting of methacrylated hyaluronic acid, and thiolated hyaluronic acid, and wherein the exosomes has a concentration in the range of 0.5-25 billion exosomes per ml of the bioengineered formulation, and wherein the primed mesenchymal stem cell derived-exosomes are exosomes derived from mesenchymal stem cells primed with corneal stromal stem cell derived-conditioned medium.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 12-48 kDa, and with a degree of substitution in the range of 20-75%; wherein the bioengineered formulation has a compressive modulus in the range of 100-1400 kPa, preferably 100-500 kPa.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 12-48 kDa, and with a degree of substitution in the range of 20-75%, wherein the bioengineered formulation is resistant to at most 50% degradation within 28 days under suitable conditions. In another embodiment of the present disclosure, the bioengineered formulation can be resistant to at most 2%, or 6%, or 8%, or 15%, or 17%, or 20%, 25, or 30%, 35%, or 40%., or 45%, or 48%.

In an embodiment of the present disclosure, there is provided a bioengineered formulation comprising: (a) a first polymer selected from the group consisting of collagen peptide or modified collagen peptide, collagen or modified collagen, and cellulose or modified cellulose; and (b) a second polymer selected from the group consisting of hyaluronic acid or modified hyaluronic acid, polyethylene glycol or modified polyethylene glycol, polyvinyl alcohol or modified polyvinyl alcohol, silk or modified silk, gelatin or modified gelatin, and alginate or modified alginate, wherein the bioengineered formulation has a compressive modulus in the range of 100-1400 kPa, preferably 100-500 kPa.

In an embodiment of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa, wherein the bioengineered formulation is resistant to at most 50% degradation within 28 days under suitable conditions.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 12-48 kDa, and with a degree of substitution in the range of 20-75%, wherein the bioengineered formulation has a transparency of at least 87%. In another embodiment of the present disclosure, bioengineered formulation has a transparency of 88-100%, or 90-98%, or 92-96%.

In an embodiment of the present disclosure, there is provided a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 12-48 kDa, and with a degree of substitution in the range of 20-75%, wherein the bioengineered formulation has an adhesive strength of at least 20kPa. In another embodiment of the present disclosure, the bioengineered formulation has an adhesive strength in a range of 21-99kPa. In another embodiment of the present disclosure, the bioengineered formulation has an adhesive strength in a range of 25-90kPa. In yet another embodiment of the present disclosure, the bioengineered formulation has an adhesive strength in a range of 40-80kPa. In one another embodiment of the present disclosure, the bioengineered formulation has an adhesive strength in a range of 50-70kPa.

In an embodiment of the present disclosure, there is provided a bioengineered formulation comprising: (a) a first polymer selected from the group consisting of collagen peptide or modified collagen peptide, collagen or modified collagen, and cellulose or modified cellulose; and (b) a second polymer selected from the group consisting of hyaluronic acid or modified hyaluronic acid, polyethylene glycol or modified polyethylene glycol, polyvinyl alcohol or modified polyvinyl alcohol, silk or modified silk, gelatin or modified gelatin, and alginate or modified alginate, wherein the bioengineered formulation has a compressive modulus in the range of 100-1400 kPa, preferably 100-500 kPa. In another embodiment of the present disclosure, the bioengineered formulation has a compressive modulus in the range of 100-1300 kPa, or 100-1000 kPa, or 100-700kPa, or 100-600kPa, or 100-300 kPa.

In an embodiment of the present disclosure, there is provided a bioengineered formulation comprising: a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa, wherein the bioengineered formulation is resistant to at most 50% degradation within 28 days under suitable conditions. In another embodiment of the present disclosure, the bioengineered formulation is resistant to at most 40% , or 30%, or 20%, or 10%, degradation within 28 days under suitable conditions.

In an embodiment of the present disclosure, there is provided a bioengineered formulation comprising: (a) a modified collagen peptide having a molecular weight in the range of 20-60 kDa, and with a degree of substitution in the range of 40-60%; and (b) a modified hyaluronic acid having a molecular weight in the range of 10-50 kDa, and with a degree of substitution in the range of 40-60%.

In an embodiment of the present disclosure, there is provided a bioengineered formulation comprising: (a) a thiolated collagen peptide having a molecular weight of 50 kDa, and with a degree of substitution of 50%; and (b) a methacrylated hyaluronic acid having a molecular weight in the range of 33 kDa, and with a degree of substitution in the range of 50%.

In an embodiment of the present disclosure, there is provided a process for obtaining a bioengineered formulation as described herein, said process comprising: (i) contacting the modified collagen peptide having a molecular weight in the range of 20-80 kDa, and with a degree of substitution in the range of 20-75% to the modified hyaluronic acid having a molecular weight in the range of 12-48 kDa, and with a degree of substitution in the range of 20-75%, to obtain a pre-mix A; and (ii) contacting the pre-mix A with a photo-initiator solution, to obtain the bioengineered formulation.

In an embodiment of the present disclosure, there is provided a process for obtaining a bioengineered formulation as described herein, said process comprising: (i) contacting the modified collagen peptide having a molecular weight in the range of 20-80 kDa, and with a degree of substitution in the range of 20-75% to the modified hyaluronic acid having a molecular weight in the range of 12-48 kDa, and with a degree of substitution in the range of 20-75%, to obtain a pre-mix A; and (ii) contacting the pre-mix A with a photo-initiator solution, to obtain the bioengineered formulation at a temperature in the range of 35-45°C, at pH 7 under dark conditions, wherein the modified collagen peptide has a concentration in the range 20-250 mg/ml with respect to the bioengineered formulation, and wherein the modified hyaluronic acid has a concentration in the range of 20-80 mg/ml with respect to the composition.

In an embodiment of the present disclosure, there is provided a process for obtaining a bioengineered formulation as described herein, said process comprising: (i) contacting the modified collagen peptide having a molecular weight in the range of 20-80 kDa, and with a degree of substitution in the range of 20-75% to the modified hyaluronic acid having a molecular weight in the range of 12-48 kDa, and with a degree of substitution in the range of 20-75%, to obtain a pre-mix A; and (ii) contacting the pre-mix A with a photo-initiator solution, to obtain the bioengineered formulation, wherein the photo-initiator solution comprises 0.05 - 0.1 mM Eosin Y and 0.038% w/v triethanolamine in phosphate buffered saline solution, and wherein the photo-initiator solution is present in an amount ranging from 0.5X-1X with respect to the bioengineered formulation. In another embodiment of the present disclosure, the photo-initiator solution comprises 0.07 - 0.09 mM Eosin Y and 0.038% w/v triethanolamine in phosphate buffered saline solution, and wherein the photo-initiator solution is present in an amount ranging from 0.6X-0.9X with respect to the bioengineered formulation.

In an embodiment of the present disclosure, there is provided a process for obtaining a bioengineered formulation as described herein, said process comprising: (i) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix A; and (ii) contacting the pre-mix with the photo-initiator solution is followed by an exposure to a white light having an intensity in the range of 50-150mW/cm² for a time period in the range of 1-15 minutes, preferably, 2-8 minutes, to obtain the bioengineered formulation.

In an embodiment of the present disclosure, there is provided a process for obtaining a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 10-100 kDa, and with a degree of substitution in the range of 20-75%; and (c) at least one type of stem cells selected from the group consisting of mesenchymal stem cells, corneal stromal stem cells, corneal limbal stem cells, and induced pluripotent stem cells, said process comprising: (i) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix A; (ii) contacting the pre-mix A with a photo-initiator solution, to obtain a pre-mix B; and (iii) contacting the pre-mix B with the at least one type of stem cells to obtain the bioengineered formulation.

In an embodiment of the present disclosure, there is provided a process for obtaining a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 10-100 kDa, and with a degree of substitution in the range of 20-75%; and (c) at least one type of stem cells selected from the group consisting of mesenchymal stem cells, corneal stromal stem cells, corneal limbal stem cells, and induced pluripotent stem cells, said process comprising: (i) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix A; (ii) contacting the pre-mix A with a photo-initiator solution, to obtain a pre-mix B; and (iii) contacting the pre-mix B with the at least one type of stem cells to obtain the bioengineered formulation, wherein the photo-initiator solution comprises 0.05 - 0.1 mM Eosin Y and 0.038% w/v triethanolamine in phosphate buffered saline solution, and wherein the photo-initiator solution is present in an amount ranging from 0.5X-1X with respect to the bioengineered formulation.

In an embodiment of the present disclosure, there is provided a process for obtaining a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 10-100 kDa, and with a degree of substitution in the range of 20-75%; and (c) at least one type of stem cells selected from the group consisting of mesenchymal stem cells, corneal stromal stem cells, corneal limbal stem cells, and induced pluripotent stem cells, said process comprising: (i) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix A; (ii) contacting the pre-mix A with a photo-initiator solution, to obtain a pre-mix B; and (iii) contacting the pre-mix B with the at least one type of stem cells is followed by an exposure to a white light having an intensity in the range of 50-150mW/cm² for a time period in the range of 1-15 minutes, preferably, 2-8 minutes, to obtain the bioengineered formulation. In another embodiment of the present disclosure, contacting the pre-mix B with the at least one type of stem cells is followed by an exposure to a white light having an intensity in the range of 60-120mW/cm² for a time period in the range of 1-10 minutes, preferably, 2-8 minutes. In yet another embodiment of the present disclosure, contacting the pre-mix B with the at least one type of stem cells is followed by an exposure to a white light having an intensity in the range of 80-100mW/cm² for a time period in the range of 2-8 minutes.

In an embodiment of the present disclosure, there is provided a process for preparing a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 10-100 kDa, and with a degree of substitution in the range of 20-75%; and (c) exosomes selected from the group consisting of corneal stromal stem cell derived-exosomes, primed mesenchymal stem cell derived-exosomes, and naive mesenchymal stem cell derived-exosomes, said process comprising: (i) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix A; (ii) contacting the pre-mix A with a photo-initiator solution, to obtain a pre-mix B; and (iii) contacting the pre-mix B with the exosomes to obtain the bioengineered formulation.

In an embodiment of the present disclosure, there is provided a process for preparing a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 10-100 kDa, and with a degree of substitution in the range of 20-75%; and (c) exosomes selected from the group consisting of corneal stromal stem cell derived-exosomes, primed mesenchymal stem cell derived-exosomes, and naive mesenchymal stem cell derived-exosomes, said process comprising: (i) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix A; (ii) contacting the pre-mix A with a photo-initiator solution, to obtain a pre-mix B; and (iii) contacting the pre-mix B with the exosomes to obtain the bioengineered formulation, wherein the photo-initiator solution comprises 0.05 - 0.1 mM Eosin Y and 0.038% w/v triethanolamine in phosphate buffered saline solution, and wherein the photo-initiator solution is present in an amount ranging from 0.5X-1X with respect to the bioengineered formulation.

In an embodiment of the present disclosure, there is provided a process for preparing a bioengineered formulation comprising: (a) a modified collagen peptide having a molecular weight in the range of 20-80 kDa, and with a degree of substitution in the range of 20-75%; (b) a modified hyaluronic acid having a molecular weight in the range of 10-100 kDa, and with a degree of substitution in the range of 20-75%; and (c) exosomes selected from the group consisting of corneal stromal stem cell derived-exosomes, primed mesenchymal stem cell derived-exosomes, and naive mesenchymal stem cell derived-exosomes, said process comprising: (i) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix A; (ii) contacting the pre-mix A with a photo-initiator solution, to obtain a pre-mix B; and (iii) contacting the pre-mix B with the exosomes is is followed by an exposure to a white light having an intensity in the range of 50-150mW/cm² for a time period in the range of 1-15 minutes, preferably, 2-8 minutes, to obtain the bioengineered formulation.

In an embodiment of the present disclosure, there is provided a bioengineered formulation comprising: (a) a first polymer selected from the group consisting of collagen peptide, modified collagen peptide, collagen, and modified collagen; (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; and (c) stem cells selected from the group consisting of mesenchymal stem cells, corneal stromal stem cells, and corneal limbal stem cells; and (d) exosomes selected from the group consisting of corneal stromal stem cell derived-exosomes, primed mesenchymal stem cell derived-exosomes, and naive mesenchymal stem cell derived-exosomes, said process comprising: (i) contacting the modified collagen peptide having a molecular weight in the range of 20-80 kDa, and with a degree of substitution in the range of 20-75% to the modified hyaluronic acid having a molecular weight in the range of 12-48 kDa, and with a degree of substitution in the range of 20-75%, to obtain a pre-mix A; (ii) contacting the pre-mix A with a photo-initiator solution, to obtain a pre-mix B; and (iii) contacting the pre-mix B with the stem cells and the exosomes, to obtain the bioengineered formulation.

In an embodiment of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; (c) stem cells selected from the group consisting of mesenchymal stem cells, corneal stromal stem cells, and corneal limbal stem cells; and (d) exosomes selected from the group consisting of corneal stromal stem cell derived-exosomes, primed mesenchymal stem cell derived-exosomes, and naive mesenchymal stem cell derived-exosomes, said process comprising: (i) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix A; (ii) contacting the pre-mix A with a photo-initiator solution, to obtain a pre-mix B; and (iii) contacting the pre-mix B with the stem cells and the exosomes, to obtain the bioengineered formulation, wherein the photo-initiator solution comprises 0.05 - 0.1 mM Eosin Y and 0.038% w/v triethanolamine in phosphate buffered saline solution, and wherein the photo-initiator solution is present in an amount ranging from 0.5X-1X with respect to the bioengineered formulation.

In an embodiment of the present disclosure, there is provided a bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; (c) stem cells selected from the group consisting of mesenchymal stem cells, corneal stromal stem cells, and corneal limbal stem cells; and (d) exosomes selected from the group consisting of corneal stromal stem cell derived-exosomes, primed mesenchymal stem cell derived-exosomes, and naive mesenchymal stem cell derived-exosomes, said process comprising: (i) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix A; (ii) contacting the pre-mix A with a photo-initiator solution, to obtain a pre-mix B; and (iii) contacting the pre-mix B with the stem cells and the exosomes is followed by an exposure to a white light having an intensity in the range of 50-150mW/cm² for a time period in the range of 1-15 minutes, preferably, 2-8 minutes, to obtain the bioengineered formulation.

In an embodiment of the present disclosure, there is provided a method for treating a corneal defect or corneal disorder in a subject, said method comprises: (a) obtaining the bioengineered formulation comprising: (i) a modified collagen peptide having a molecular weight in the range of 20-80 kDa, and with a degree of substitution in the range of 20-75%; and (ii) a modified hyaluronic acid having a molecular weight in the range of 10-100 kDa, and with a degree of substitution in the range of 20-75%; (b) applying a suitable amount of the bioengineered formulation at the site of a corneal defect; and (c) illuminating a white light having an intensity in the range of 50-150mW/cm² on the formulation at the site of the corneal defect for a time period in a range of 1-15 minutes, preferably, 2-8 minutes, for treating the corneal defect in a subject, for treating the corneal defect in a subject. In another embodiment of the present disclosure, illuminating a white light having an intensity in the range of 70-100mW/cm² on the formulation at the site of the corneal defect for a time period in a range of 5-10 minutes, preferably, 2-8 minutes, for treating the corneal defect in a subject, for treating the corneal defect in a subject.

In an embodiment of the present disclosure, there is provided a method for treating a corneal defect or corneal disorder in a subject, said method comprises: (a) obtaining the bioengineered formulation comprising: (i) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the formulation has a compressive modulus in the range of 100-1400 kPa, preferably 100-500 kPa; (b) applying a suitable amount of the bioengineered formulation at the site of a corneal defect; and (c) illuminating a white light having an intensity in the range of 50-150mW/cm² on the formulation at the site of the corneal defect for a time period in a range of 1-15 minutes, preferably, 2-8 minutes, for treating the corneal defect in a subject, for treating the corneal defect in a subject.

In an embodiment of the present disclosure, there is provided a method for treating a corneal defect or corneal disorder in a subject, said method comprises: (a) obtaining the bioengineered formulation as described herein; (b) applying a suitable amount of the bioengineered formulation at the site of a corneal defect; and (c) illuminating a white light having an intensity in the range of 50-150mW/cm² on the formulation at the site of the corneal defect for a time period in a range of 1-15 minutes, preferably, 2-8 minutes, for treating the corneal defect in a subject, for treating the corneal defect in a subject, and wherein the method further comprises applying a solution comprising: (i) exosomes selected from the group consisting of corneal stromal stem cell derived-exosomes, primed mesenchymal stem cell derived-exosomes, and naive mesenchymal stem cell derived-exosomes; and (ii) a clinically approved eye drop formulation, at the site of the corneal defect before or after applying the suitable amount of the bioengineered formulation.

In an embodiment of the present disclosure, there is provided a formulation comprising: (a) exosomes selected from the group consisting of corneal stromal stem cell derived-exosomes, primed mesenchymal stem cell derived-exosomes, and naive mesenchymal stem cell derived-exosomes; and (b) a clinically approved eye drop formulation, wherein the eye drop formulation comprises 0.1-0.25% hyaluronic acid. In another embodiment of the present disclosure, the eye drop formulation comprises 0.2-0.22% hyaluronic acid

In an embodiment of the present disclosure, there is provided a method for treating a corneal defect in a subject, said method comprising: obtaining a formulation comprising: (i) exosomes selected from the group consisting of corneal stromal stem cell derived-exosomes, primed mesenchymal stem cell derived-exosomes, and naive mesenchymal stem cell derived-exosomes; and (ii) a clinically approved eye drop formulation, wherein the eye drop formulation comprises 0.1-0.25% hyaluronic acid; and (b) applying the formulation at the site of the corneal defect, for treating the corneal defect in a subject.

In an embodiment of the present disclosure, there is provided a bioengineered formulation as described herein, for use in treating a corneal defect in a subject.

In an embodiment of the present disclosure, there is provided a formulation as described herein, for use in treating a corneal defect in a subject

Although the subject matter has been described in considerable detail with reference to certain examples and implementations thereof, other implementations are possible.

### EXAMPLES

The disclosure will now be illustrated with working examples, which is intended to illustrate the working of disclosure and not intended to take restrictively to imply any limitations on the scope of the present disclosure. Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein. It is to be understood that this disclosure is not limited to particular methods, and experimental conditions described, as such methods and conditions may apply.

### MATERIALS AND METHODS

### SOURCE OF STEM CELLS

For the purpose of the present disclosure, the source of stem cells includes derived from the sources such as human bone marrow (BM), corneal limbal stem cells (CLSC), umbilical cord (UC), Wharton's jelly (WJ), dental pulp (DP) and adipose tissue (AD), corneal limbal stem cell-derived conditioned media primed MSCs (CLSC-CM primed MSCs) can be used in the methods and cell-derived products as described herein. The choice of the stem cell type would be target indication and tissue specific.

### Source of immortalized adult stem cell lines (Non-Viral immortalized MSC cell lines):

1. Telomerized human Bone marrow derived mesenchymal stem cell line (BMMSC/TERT277) was developed from mesenchymal stem cells isolated from spongy bone (sternum) by non-viral gene transfer of a plasmid carrying the hTERT gene. Positively transfected cells were selected by using neomycin phosphotransferase as selectable marker and Geneticin sulfate addition. The cell line was continuously cultured for more than 25 population doublings without showing signs of growth retardation or replicative senescence.
2. Telomerized human Wharton's Jelly derived mesenchymal stem cell line (WJ-MSC/TERT273) was established under xeno-free conditions from primary tissue disaggregation to non-viral transfer of hTERT.

The cell lines were characterized by unlimited growth while maintaining expression of cell type specific markers and functions such as: (i) typical mesenchymal morphology; (ii) expression of typical mesenchymal stem cell markers such as CD73, CD90 and CD105; (iii) differentiation potential towards adipocytes, chondrocytes, osteoblasts; and (iv) production of extracellular vesicles with angiogenic and anti-inflammatory activity.

### Method of culturing stem cells and./or cell derived products

The present disclosure discloses process for culturing cells for generation of cells, and cell derived products such as secretome, exosomes, extracellular matrix components (ECM) and other cell derived-components of medical interest, including but not restricted to regenerative treatment of various diseases including inflammatory or fibrotic conditions of tissues/organs of liver, lung, pancreas, kidney, cornea, heart and brain.

Stromal/Stem cells from various sources like human Bone Marrow derived Mesenchymal Stem Cells (BMMSC) or human donor derived Corneal limbal Stem Cells (CLSC) were cultured in 3 different methods, namely the two-dimensional 2D, a three-dimensional (3D) micro-sphere based and 3D spheroid culture under xenofree conditions. The conditioned media from these cultures were characterized for the secretome and exosome fractions and therapeutically beneficial components were identified. The detailed process for culturing the bone Marrow derived Mesenchymal Stem Cells (BMMSC) or human donor derived Corneal limbal Stem Cells (CLSC), or obtaining the CLSC-CM primed MSCs, by three different methods, namely the 2D, a 3D micro-sphere based and 3D spheroid culture under xenofree conditions are described in the pending applications PCT/IN2020/050622, and PCT/IN2020/050623 which are incorporated in its entirety in the present disclosure.

### Source of the components used in the bioengineered formulation

The two major polymers of the bioengineered formulation thiolated hyaluronic acid (HA-MA) and thiolated recombinant collagen peptide (RCP-SH) were procured from Creative PEG works, Fujifilm, respectively.

### EXAMPLE 1

### BIOENGINEERED FORMULATIONS

The present example discloses a bioengineered formulation comprising two major components- modified hyaluronic acid and modified collagen peptide. Particularly, in the present disclosure, the modified hyaluronic acid is a methacrylated hyaluronic acid (HA-MA), whereas the modified collagen peptide is methacrylated recombinant collagen peptide (collagen type I-based peptide or RCP) (RCP-MA), or thiolated recombinant collagen peptide (RCP-SH). Apart from the afore-mentioned components, the bioengineered formulation also comprises photo initiator solution (0.001-0.1 mM Eosin Y and 0.038% w/v triethanolamine in phosphate buffered saline (PBS) solution PBS solution) with which the components can be photo-crosslinked with short time exposure to white light. The bioengineered formulation is a solid and transparent hydrogel that firmly adheres to the corneal tissue, and can be used for non-invasive, quick and long-term repair of corneal stromal defects.

For the purpose of the present disclosure, the physical properties of the bioengineered formulation were tuned to mimic those of the native cornea. In order to ascertain that the properties of the bioengineered formulation are comparable to the properties of the native cornea, the following set of parameters were analysed: i. Compressive Modulus in the range of 100-300 kPa; ii. Adhesion Strength: >20 kPa; iii. Transparency-Target value: >87%; iv. *Ex-vivo* burst pressure: >2.5 kPa (nominal intraocular pressure of human eye); v. Pot life; vi. Crosslinking kinetics; vii. Swelling profile: <35%; viii. Biocompatibility - *In-vitro* studies; and ix. Safety and Efficacy *- In-vivo* studies in a rabbit model. The terms bioengineered formulation and hydrogel formulations are used interchangeably.

The detailed analysis of the afore-mentioned parameters on which the physical properties of the bioengineered formulation were assessed are provided below:

### (A) Compressive Modulus and Adhesion Strength

To check the effect of increase or decrease in HA-MA molecular weight on the compressive modulus and adhesion strength, the bioengineered formulations with varied molecular weight of HA-MA were screened. The screening of the three bioengineered formulations was done by varying the molecular weight of HA-MA. The molecular weight of HA-MA is one of the important parameters for accessing the physical, mechanical, and other functional properties of the bioengineered formulation of the present disclosure. Therefore, the screening of the three bioengineered formulations was done with 33kDA HA-MA, 10kDA HA-MA, 50kDa HA-MA, RCP-SH / RCP-MA. In all the examples, the "33 kDa" HA-MA and RCP-SH hydrogel formulation refers to 33 kDa of HA-MA and 50 kDa of RCP-SH and the concentrations may vary as per the experiment as described herein.

### Experiments with "33 kDa" HA-MA and RCP-SH hydrogel formulation

The first set of screening was initiated with the "33 kDa" HA-MA, RCP-MA and RCP-SH formulations, with degree of substitution of 50%. The RCP-SH used in the present Example is of 50 kDa molecular weight. As shown in Figure 1A and 1B, the compressive modulus and adhesion strength of the single component hydrogel made using HA-MA 100 mg/ml (i.e., without RCP-MA/RCP-SH) was found to be lower than the two component hydrogels suggesting that addition of - MA or -SH groups increase the intermolecular crosslinking and have a significant effect on the physical properties of the hydrogel. The single component solution using RCP-MA (125 and 250 mg/ml) (the single component as mentioned herein is without HA-MA or RCP-SH) did not gel after exposure to white light, when eosin was used as photo initiator. Similarly, the RCP-SH (125 and 250 mg/ml) solutions also did not form gel on exposing to blue light in the presence of riboflavin as photo initiator. Keeping the concentration of RCP-MA and RCP-SH constant (125 mg/ml) and varying the HA-MA concentrations (from 10 to 100 mg/ml), it was observed that with increase in the concentration of HA-MA, the compressive modulus of hydrogels also increases. The hydrogel formulations with 10 mg/ml HA-MA represented the lower value of the compressive modulus, whereas the formulation having 50 mg/ml or above showed higher value of compressive modulus than the target value range (~300 kPa) of the mechanical strength. The compressive modulus of the hydrogel formulation with 35 or 40 mg/ml HA-MA was found to be in the required range (100-300 kPa). Moreover, it was also observed that the compressive modulus was found to be directly proportional to the RCP-SH concentration. When the compressive modulus of RCP-MA was compared with RCP-SH, for same concentration of HA-MA, it was found out that the compressive modulus of hydrogels having RCP-SH was higher than the hydrogels comprising same amount of RCP-MA. It can also be observed from Figure 1A that the compressive modulus of the hydrogel formulation (comprising HA with molecular weight of "33kDa" and concentration of "40 mg/ml" and RCP-SH with "125 mg/ml") was higher than the Gel-MA(in-house).

Referring to Figure 1B, the adhesion strength of the hydrogel formulations increased with an increase in HA-MA concentration. The effect of increasing the RCP-SH was further assessed that didn't show significant change in the adhesion strength. However, the adhesive strength of the hydrogel formulation of the present disclosure was comparable with the Gel-MA (in-house).

It can be inferred from the above observations, that the hydrogel formulation with HA-MA of molecular weight "33 kDa" and concentration of "40mg/ml" and RCP-SH with 125 mg/ml that exhibited desired compressive modulus and adhesive strength value, was further screened to confirm the working formulation of the present disclosure.

### Experiments with "10 kDa" HA-MA, RCP-SH hydrogel formulation

Post-screening for "33 kDa" HA-MA formulations, it was necessary to check the effect of increase/decrease in HA-MA molecular weight on the compressive modulus and adhesion strength. Since change in HA-MA concentration did not appear to have a significant effect on the adhesion strength, further screenings were performed by varying the concentration of RCP-SH. As shown in Figure 2A and 2B, the screening studies were done with "10 kDa" HA-MA, RCP-MA and RCP-SH formulations, with degree of substitution of 30%. The screening studies as shown in Figure 2, reveal that in the hydrogel formulation with molecular weight of 10 kDa and concentration of HA-MA with 150 mg/ml, the compressive modulus and adhesive strength increased with the increase in RCP-SH concentration. Further, it can be observed that compressive modulus of the hydrogel formulation was 4-7.5 times higher than the reported value of 20% w/v of Gel-MA (Shirzaei Sani E, Kheirkhah A, Rana D, Sun Z, Foulsham W, Sheikhi A, Khademhosseini A, Dana R, Annabi N. Sutureless repair of corneal injuries using naturally derived bioadhesive hydrogels. Sci Adv. 2019 Mar 20;5(3):eaav1281. doi :10.1126/sciadv. aav1281) (Figure 2A). Similarly, the adhesion strength of the hydrogels was >2.0 times higher than the reported value of Gel-MA and Fibrin Glue (Nasim Annabi, Suzanne M. Mithieux, Pinar Zorlutuna, Gulden Camci-Unal, Anthony S. Weiss, and Ali Khademhosseini. Engineered cell-laden human protein-based elastomer. Biomaterials. 2013 Jul; 34(22): 5496-5505. doi: 10.1016/j.biomaterials. 2013.03.076) (Figure 2B). The values obtained for the 10 kDa formulations were also significantly greater than the "33 kDa" formulations. However, the highest achievable concentration of HA-MA of 10 kDa HA-MA was 150 mg/ml beyond which the solution became highly viscous which blocked the pipette/needle tip. Moreover, the presence of HA-MA at a concentration of 150 mg/ml made the hydrogel formulation very unstable, and 150 mg/ml concentration solution of HA-MA solution started self-crosslinking in ambient light with and without the photo initiator. Therefore, it can be inferred from Figure 2A and 2B that along with the presence of HA-MA at a desired molecular weight of "33 kDa", the concentration of HA-MA in the hydrogel formulation in the range of 20-80mg/ml is highly important for obtaining a stable, viscous, and biocompatible hydrogel formulation of the present disclosure. Any deviation in the concentration of the HA-MA from the disclosed range (20-80mg/ml) make the hydrogel formulation highly viscous and unstable.

### Experiments with "50 kDa" HA-MA, RCP-SH hydrogel formulation

Having established the effect of molecular weight, concentration of HA-MA and concentration of RCP-SH, the effect of increase in HA-MA molecular weight was further tested by screening the hydrogel formulation comprising HA-MA with molecular weight of 50 kDa for compressive and adhesion strength. The hydrogel formulation with 50 kDa comprised 75 mg/ml of HA-MA and 125 mg/ml of RCP-SH. The screening results of the hydrogel formulation with 50 kDa HA-MA are shown in Figure 3A and Figure 3B. Although the hydrogel formulation with 50 kDa of HA-MA exhibited compressive modulus of 1379.8 kDa (Figure 3A), and adhesive strength of 70 kPa (Figure 3B), however, the hydrogel formulation containing 75 mg/ml of HA-MA with molecular weight of 50 kDa and RCP-SH with concentration of 125 mg/ml, was too viscous and required the aid of vortex mixer or other equipment for homogenous mixing of the components. Therefore, hydrogel formulation comprising HA-MA with molecular weight of 50 kDa was not further screened.

### Rationale for selecting "33 kDa" HA-MA, RCP-SH over "10KDa HA-MA", RCP-SH formulation and "50 kDa" HA-MA, RCP-SH hydrogel formulation

Overall, it can be inferred from Figures 1-3, that although the hydrogel formulation with 10 kDa HA-MA had low viscosity even at concentrations as high as 150 mg/ml; however, due to their instability (tendency to self-crosslink in the absence of photo-initiator), the clinicians might find difficulty in handling the said formulation. Similarly, 50 kDa HA-MA formulations were very viscous and difficult to handle with increasing concentrations leading to limitations in the highest achievable modulus and adhesive strength. In contrast, the "33 kDa" HA-MA formulations provided balance between stability and handling among various formulations. Therefore, the "33 kDa" HA-MA/RCP-SH formulations were chosen for further characterization with burst pressure and pot-life studies.

### (B) Transparency

The 33 kDa HA-MA/RCP-SH formulations comprising HA-MA and RCP-SH at various concentrations were screened for transparency. Transmittance values were obtained by recording the absorbance of the samples in the range of 350-750 nm, using saline as blank. The obtained absorbance (A) values were converted to transmittance (%T) using Beer Lambert's law. According to Beer-Lambert's Law, %T=10^{(2-Absorbance)} (according to the protocol described in Wang et al., 2015. Biomacromolecules 2014, 15, 9, 3421-3428. https://doi.org/10.1021/bm500969d)

For this purpose, the transmittance (%) of 33 kDa HA-MA/RCP-SH formulations were compared with Gel-MA (20%). As shown in Figure 4, all the hydrogel formulations irrespective of the components, their molecular weight and proportions indicated ~80% transmittance relative to 1X PBS. In particular, the formulations comprising HA-MA at a concentration of 35 mg/ml or higher, showed light transmittance >87%.

### (C)Ex-vivo burst pressure

Figure 5 shows the *ex-vivo* burst pressure tested for "33 kDa" HA-MA/RCP-SH and Gel-MA (20% w/v, DoS >95%) formulations. It can be observed from Figure 5 that the *ex-vivo* burst pressure varied across the changes in the concentration of HA-MA and RCP-SH present in the formulations. However, statistically (alpha = 0.05), the values did not differ significantly. Nonetheless, the burst pressure sustained by bioengineered formulation was significantly higher than the intra-ocular pressure (IOP). As shown in Figure 5, the IOP value of the bioengineered formulation having 30mg/ml of HA-MA and 125mg/ml of RCP-SH was 2 times higher than the IOP of native cornea (~2.5 kPa). Further, it can be observed that the IOP value of the bioengineered formulation having 40 mg/ml of HA-MA and 125mg/ml of RCP-SH was 10-13 times higher than the IOP of native cornea. Moreover, the ex-vivo burst pressure values of bioengineered formulation having 75 mg/ml or a higher concentration of HA-MA were significantly higher than ex-vivo burst pressure value of the commercially available adhesive (Fibrin glue; 21.7 kPa) or previously reported corneal adhesive (Gel-MA 20% w/v, DoS >95%; 30.1 kPa).

### (D)Pot life

This example highlights the importance of the concentration of the photo-initiator that can be added in the hydrogel formulation of present disclosure. The thiol-ene crosslinking process was accelerated by the addition of a photo-initiator like eosin. Moreover, eosin mediated photo initiation was activated in the presence of white light. Although high intensity-white light is required to crosslink the hydrogel adhesive in 2 mins, ambient light can start the crosslinking process making it difficult for the clinician to handle the formulation in the process of applying it on the corneal defect.

Figure 6A shows the hydrogel formulation prepared by reconstituting and mixing the solutions of the components (33kDa HA-MA with 125 mg/ml, RCP-SH with 150 mg/ml, and 1X photo initiator), separately. It can be observed from Figure 7A that the hydrogel formulation showed steady increase in storage modulus and complex viscosity after ~5 mins exposure to ambient light. In contrast, the same hydrogel formulation with the components (33kDa HA-MA with 125 mg/ml, RCP-SH with 150 mg/ml, and 1X photo initiator) was prepared by using a pre-mixed protocol as shown in Figure 6B, wherein the components were pre-mixed in powder form and then were reconstituted in saline solution. It can be observed from Figure 7B that the hydrogel formulation had a higher storage modulus and complex viscosity at t = 0. In addition, the storage modulus and complex viscosity values started increasing steadily within 2 mins of exposure to ambient light (Figure 7).

Further, referring to Figure 7A, all hydrogel formulations prepared with 0.5X concentration of the photo initiator, irrespective of the protocol employed, remained stable without any crosslinking for up to 20 mins. Overall, it can be inferred from Figure 6 and 7, that when the concentration of the photo initiator was reduced to half, the pot-life of the hydrogel formulations increased significantly. The increase in pot-life of the hydrogel formulation would give sufficient time to the clinicians and helping staff to prepare and apply the formulation at the site of the corneal defect.

### (E) Crosslinking kinetics

Figure 8 shows the comparison of the cross-linking kinetics of the 33 kDa HA-MA/RCP-SH hydrogel formulations (30 mg/ml of HA-MA/ 125mg/ml of RCP-SH, 40 mg/ml of HA-MA/ 125mg/ml of RCP-SH, 50 mg/ml of HA-MA/ 125mg/ml of RCP-SH; with degree of substitution of 50%) with the cross-linking kinetics of 20%w/v of Gel-MA formulation. The crosslinking kinetics data as shown in Figure 8 demonstrated that storage modulus, which directly relates to the completion of the crosslinking reaction of HA-MA/RCP-SH system, attained equilibrium within 5 min, whereas, Gel-MA formulation did not attain equilibrium even after 15-30 min. Furthermore, HA-MA/RCP-SH formulations demonstrated a 100-fold increase in storage modulus compared to single component Gel-MA formulation. Hence, the bioengineered formulations of the present disclosure is preferable than the well-known methacrylate gelatin (Gel-MA) hydrogel.

### (F) Swelling profile

The swelling study was performed by incubating the 33 kDa HA-MA/RCP-SH hydrogel formulation in 1X PBS for 48 h. Figure 9 shows the comparison of the swelling profile of 33 kDa HA-MA/RCP-SH hydrogel formulation with different polymer concentrations with the swelling profile of Gel-MA formulations. The results of the swelling study as shown in Figure 9 demonstrated that 33 kDa HA-MA/RCP-SH hydrogel formulation attained equilibrium within 4 h, whereas Gel-MA formulations did not attain equilibrium until 24 h. Furthermore, 33 kDa HA-MA/RCP-SH hydrogel formulations demonstrated a very controlled swelling as compared to Gel-MA formulation, wherein Gel-MA formulation showed high fluid uptake initially, and the fluid uptake then kept on increasing until a period of 72 h. Rate of swelling of the hydrogel formulations of the present disclosure decreased with increase in polymer concentration. The hydrogel formulation having HA-MA at a concentration of 35 mg/ml or a higher concentration demonstrated ~50% lesser swelling than the other formulations. Therefore, the hydrogel formulation of the present disclosure with the lesser swelling profile are more preferred for therapeutic applications or the like.

### (G) Biodegradation

Hydrogels of definite volume were prepared, lyophilized and weighed (Wi). Replicate hydrogels were then incubated in PBS or saline (pH~7.4) at 37 °C and shaken in orbital shaker. At specific time points, hydrogels were taken out, lyophilized and weighed (Wd). Then mass loss was calculated as: Weight loss or degradation (%) = (Wi-Wd)/Wi x 100 (Li 2006, Biomaterials https://dx.doi.org/10.1016%2Fj.biomaterials.2005.07.019)

Figure 10 shows the biodegradation profile of 33 kDa HA-MA/RCP-SH hydrogel formulations (with 50% degree of substitution), compared with respect to time.

Referring to Figure 10, the degradation rate of the hydrogel formulations increased with increase in the concentration of HA-MA. The rate of degradation of hydrogel formulation was steady until day 14. However, after day 14, the hydrogel formulation with higher polymer content (i.e., hydrogel formulation comprising HA-MA at a concentration of 40 mg/ml and RCP-SH at a concentration of 150mg/ml) showed sudden increase with maximum of 52.87% and 46.36% degradation on day 28. Therefore, it can be inferred from Figure 10, that the 33 kDa HA-MA/RCP-SH hydrogel formulations demonstrated controlled degradation as compared to Gel-MA which disintegrated within a period of 12 h (Gel-MA data not shown).

Table 1 provides a summary of the physical properties of the bioengineered formulations tuned to mimic those of the native cornea.

**Table 1**

| **Formulation No.** | **HA-MA (mg/ml)** | **RCP-SH (mg/ml)** | **Compressive Modulus (kPa)** | **Optical Clarity (%)** | **Equilibrium Swelling (%)** | **Burst Pressure (kPa)** | **Adhesive Strength (kPa)** | **Degradation (%) (28 days)** |
|---|---|---|---|---|---|---|---|---|
| **Native cornea** | | | **100-300** | **87-94** | **<35%** | **>2.5** | **>20** | **< 50** |
| 1 | 20 | 125 | 144.31 ± 27 | | 30.13 | | 26.33 ± 18.21 | |
| 2 | 25 | 125 | 201.48 ± 16.13 | 79.27 | - | - | - | - |
| 3 | 25 | 150 | 244.31 ± 22.12 | 79.37 | 31.15 | - | - | - |
| 4 | 30 | 125 | 248.08 ± 24.7 | 81.51 | 26.04 | 6.8 ± 1.9 | 42.13 ± 6.4 | 16.78 |
| 5 | 30 | 150 | 216.04 ± 5.13 | 83.16 | - | - | - | - |
| 6 | 35 | 125 | 203.06 ± 35.48 | 87.74 | 17.01 | 27.47 ± 7.68 | 32.38 ± 4.73 | 8.01 |
| 7 | 35 | 150 | 294.87 ± 19.58 | 87.73 | 12.655 | 27.87 ± 5.4 | 44.03 ± 13.33 | 17.29 |
| 8 | 35 | - | Too brittle | - | 34.21 | - | - | - |
| 9 | 40 | 100 | 238.89 ± 11.42 | - | - | - | - | - |
| 10 | 40 | 125 | 368.44 ± 61 | 89.78 | 16.2 | 33.6 ± 5.02 | 34.22 ± 9.57 | 39.09 |
| 11 | 40 | 150 | 340.08 ± 29.7 | 89.80 | 14.19 | 25.6 ± 0.2 | 31.62 ± 12.36 | 46.36 |
| 12 | 50 | 125 | 651.39 ± 189 | - | - | - | - | - |
| 13 | 75 | 125 | 782.32 ± 50 | 91 | 12.82 | 44.05 ± 4.7 | 114.14 ± 20 | 31.98 (day 14) |
| 14 | 75 | 150 | 1339 ± 50 | 91.33 | 9.0 | 50.1 ± 2.0 | 110.9 ± 11 | |
| 15 | 100 | 125 | 2430.5 ± 220 | - | - | - | - | - |
| 16 | Gel-MA 20%, DoS >95% (in-house) | | 164.72 ± 12 | 85.94 | 74 | Flows down | 28 ± 3.1 | >90 |
| 17 | Gel-MA 20%, DoS 65-80% (Ref. 1) | | 299.9 ± 30 | - | - | 30.1 ± 4.3 | 90.4 ± 10.2 | - |
| 18 | Fibrin (in-house) | | - | - | - | 21.7 ± 3 | 105.04 ± 44 | 92 |

Referring to Table 1, it can be concluded that the presence of the components, i.e., HA-MA (modified hyaluronic acid), RCP-SH (modified collagen peptide) in the disclosed ranges is important for obtaining the hydrogel formulation that exhibits desired physical properties which can be tuned to mimic the physical properties of the native cornea. Considering this, the absence of RCP-SH in the formulation 8 makes the hydrogel formulation very brittle, and therefore, the formulation 8 which does not show the desired physical properties is considered as a non-working formulation. Additionally, the presence of the HA-MA at a concentration of 100 mg/ml which is outside the disclosed concentration range (20-80 mg/ml) makes the formulation 15 as another non-working formulation since the formulation has a very high compressive modulus which is not preferable. It can be inferred from Table 1 that the presence of HA-MA, and RCP-SH at the disclosed ranges is critical for obtaining the hydrogel formulation of desirable physical properties. Further, it can also be observed that the bioengineered formulations of the present disclosure perform better than the well-known Gel-MA hydrogel. Therefore, the formulations 1-7 and 9-14 are the working formulations of the present disclosure, and formulations 8, 15-18 are the non-working formulations.

### EXAMPLE 2

### PROCESS OF PREPARING THE BIOENGINEERED FORMULATION

The present example describes the optimized process for preparing the bioengineered formulation. The steps for preparing the formulation are depicted in Figure 11, and are also described below:
(i) Vial I containing the lyophilized bioengineered cornea powder (comprising a combination of 33 kDa HA-MA having a concentration in the range of 2-7.5mg and RCP-SH having a concentration in the range of 5-15 mg/ml) and vial II containing the photo-initiator solution (100µl) was warmed to a root temperature (RT) protected from light.
(ii) Using a needle and syringe, the photo-initiator solution from vial II was transferred to the contents of vial I protected from light. Vial I was then gently swirled to ensure that the contents were soaked to promote dissolution and was then incubated at 37°C for 30 min under dark condition.
(iii) The components of vial I were fully dissolved to ensure that the final pre-hydrogel formulation was homogenous in nature.
(iv) Using a needle and syringe, the required volume was administered on to the cornea defect site and the pre-hydrogel formulation was exposed to white light for 5 min.
(v) Post-exposure to white light, the defect site was irrigated with saline solution to hydrate the crosslinked hydrogel.

### METHOD OF TREATMENT USING THE BIOENGINEERED FORMULATION

The volume of bioengineered formulation dispensed at the site of corneal defect depends on the volume of the corneal scar and the discretion of the clinician.

For instance, the average volume, accounting for 15% hydrogel swelling, recommended for scars of definite size is given in the Table 2 below.

**Table 2: Volume of the hydrogel formulation to be dispensed for corneal scars. Scars are assumed to be perfect cylinders for the calculations.**

| | **Diameter (mm)** | **Depth (µm)** | | |
|---|---|---|---|---|
| **S.No.** | **Wound dimensions** | | **Wound volume (mm³)** | **Volume of Bioengineered formulation (bioengineered cornea) to be dispensed (µL)** |
| | **Diameter (mm)** | **Depth (µm)** | | |
| 1. | 5 | 100 | 1.96 | 2.3 |
| 2. | 5 | 150 | 2.95 | 3.4 |
| 3. | 5 | 200 | 3.93 | 4.5 |
| 4. | 5 | 250 | 4.91 | 5.7 |
| 5. | 5 | 300 | 5.89 | 6.8 |
| 6. | 6 | 100 | 2.83 | 3.3 |
| 7. | 6 | 150 | 4.24 | 4.9 |
| 8. | 6 | 200 | 5.65 | 6.5 |
| 9. | 6 | 250 | 7.07 | 8.1 |
| 10. | 6 | 300 | 8.48 | 9.8 |

### FINAL BIOENGINEERED FORMULATION

The bioengineered formulation comprising HA-MA having a concentration in the range of 20-75 mg/ml with molecular weight of "33 kDa", and RCP-SH of molecular weight 50 kDa having a concentration in the range of 20-250 mg/ml, and a photo-initiator (eosin) having a concentration of 0.5X was selected as the final formulation along with the optimized protocol as provided in Figure 11 that would be followed by the clinicians to treat the corneal defects or corneal disorders.

### EXAMPLE 3

### BIOCOMPATIBILITY OF THE BIONEGINEERED FORMULATION: IN-VITRO STUDIES

The bioengineered formulations as explained were assessed for their suitability to elicit corneal tissue regeneration. Firstly, the re-epithelialization capability using the limbal or corneal epithelial cells (LECs or CECs) on hydrogel surfaces were studied. Secondly, to demonstrate stromal regeneration, corneal limbal stem cells (CLSCs) were encapsulated inside the hydrogels and their viability, proliferation capacity and phenotype were studied *in-vitro.*

### (i) Re-epithelialization study

To demonstrate biocompatibility of the hydrogel formulation ("33 kDa" HA-MA/RCP-SH hydrogel formulations (comprising 33 kDa of HA-MA/ 50 kDa of RCP-SH in the concentrations of 75/125mg/ml and 75/150 mg/ml) of the present disclosure, primary human CECs were seeded and cultured on the surface. The epithelial cells adhered and proliferated on the surface of the hydrogels yielded a confluent monolayer by the end of two weeks, as shown in Figure 12. This observation was comparable to the 2D coverslip surface and Gel-MA (20% w/v) hydrogel, which were used as positive controls. It can be concluded from Figure 12 that hydrogel formulations of the present disclosure act as a cornea-mimetic bioengineered material that can promote corneal wound healing/ regeneration *in-vivo.*

### (ii) Stromal Regeneration: Encapsulation of CLSCs in the hydrogel formulation

The present example demonstrates the effect of the combination of the hydrogel formulation and stem cells for treating the corneal disorders. For this purpose, the stem cells, such as, CSSC were encapsulated in the hydrogel formulation. The compatibility of the hydrogels to the CLSCs, which would ultimately indicate the stromal regeneration capability of the hydrogel, was assessed by culturing CLSCs on the hydrogel surface followed by encapsulation studies.

Figure 13 (A-H) shows the viability assessment of the CLSCs when cultured on the hydrogel surface for 5 days. As evident from the results, the CLSCs showed rapid proliferation and covered the hydrogel surface within 5 days. Further, the viable cell population, marked by cell cytoplasm stained in green color, shows that the culture environment provided by the hydrogel formulation is compatible for the cells to proliferate. Overall, it can be observed from Figure 13, that the cell growth on the hydrogel formulation surface, was higher than that on 20% of Gel-MA (Figure 13 C and Figure 13G), whereas, it was similar to the cells on coverslips which was used as a positive control (Figure 13D and Figure 13H).

The viability of CLSCs was also assessed for 2 weeks on encapsulating the cells in the 33kDa HA-MA/RCP-SH hydrogel formulations comprising HA-MA (33 kDa) /RCP-SH (50 kDa) in the ratios of 75/125mg/ml, 75/150 mg/ml, and 40/125 mg/ml. As shown in Figure 14, the cells that appeared green (due to calcein-AM stain taken up by live cells), represented the live cell population which showed ~95% cell viability. The CLSCs encapsulated in the hydrogel formulations of the present disclosure were viable throughout the culture duration, and the viable population was similar to the 2D cover slip and was higher than the Gel-MA (20%; DOS >95%). Figure 14B shows an enlarged image on day 14 of the hydrogel formulation comprising HA-MA /RCP-SH in the ratio of 40/125mg/ml. It can be observed from Figure 14C that majority of the cells attained an elongated morphology. The similar kind of elongated morphology was also shown by cells cultured on 2D surface. The 3D reconstruction of ~40 layers (minimum) of the images also confirmed the homogenous distribution of viable cells within the hydrogel formulation (Figure 14D).

For complete tissue regeneration at the defect site, it is of utmost importance that the stromal stem cells maintain their phenotype and help in scar-less healing of the wound while gradually attaining the differentiated state. In *in-vitro* condition, this process of gradual differentiation can be assessed by checking the expression of biomarkers which are specific to a particular stage of cell's life cycle. CD90 is one such biomarker which is expressed by the stromal stem cells, whereas the expression of αSMA by the cells would reflect their differentiated state to keratocytes or myofibroblasts. Figure 15 depicts the immunofluorescence study showing expression of CD90 (red) and αSMA (green) by the CLSCs encapsulated in the hydrogel formulation with respect to 2D culture surface. As shown in Figure 15, CLSCs cultured in the hydrogel formulations of the present disclosure (comprising HA-MA/RCP-SH in the ratios of 40/125 mg/ml, and 75/125 mg/ml; DOS 50%), showed better expression of CD90 and did not express αSMA. In contrast, the cells cultured on the 2D surface showed prominent expression of αSMA, indicating their differentiated phenotype. Therefore, it can be inferred from Figure 15, that the hydrogel formulations of the present disclosure suppressed myofibroblast differentiation and hence have the potential to support scar-less wound healing of the corneal tissue.

### EXAMPLE 4

### BIOCOMPATIBILITY OF THE BIONEGINEERED FORMULATION: IN-VIVO STUDIES

To demonstrate safety and efficacy of the hydrogel formulations of the present disclosure *in-vivo,* hydrogel formulation (33 kDa HA-MA/ 50 kDa RCP-SH present at 75/150 mg/ml; both with DOS 50%) was applied to a clinically-relevant rabbit model of corneal injury. Briefly, rabbits were anesthetized and corneal stromal injuries were introduced with trephine blade and a wound with 7 mm in diameter and 250 µm in average depth was created in central region of the cornea. After surgery, rabbits either received clinical grade tissue adhesive, cyanoacrylate glue, used in standard of care for corneal perforation, or hydrogel formulation of the present disclosure.

Figure 16 shows the pre-clinical study of hydrogel formulations (bioengineered formulation of the present disclosure is referred to as bioengineered corneal v1.0 in the Figure 16) in rabbit model *in-vivo.* As shown in Figure 16, rabbit corneas receiving treatment with cyanoacrylate glue did not demonstrate a transparent cornea, whereas rabbit corneas receiving hydrogel formulations of the present disclosure exhibited corneal transparency by the end of 2 weeks. Similar results were observed for fluorescein staining, an indicator of epithelial healing, and densitometry scans, a standard clinical evaluation method for measuring corneal transparency. Rabbit corneas receiving cyanoacrylate glue treatment demonstrated fluorescein pooling and severe corneal opacity due to presence of scar tissue, whereas rabbit corneas receiving the hydrogel formulation of the present disclosure exhibited a gradual decrease in dye pooling and demonstrated gradual improvement in corneal transparency and vision over the period of 2 weeks.

It can be inferred from Figure 16, that application of cyanoacrylate glue in rabbit cornea wound (Figure 16A) caused corrosive irregular surface of cornea, which becomes opaque and attracts blood vessels (indicated with red arrow). On the other hand, the application of the hydrogel formulation of the present disclosure (Figure 16B) showed gradual gain on transparency, no angiogenesis and completely healed epithelium with smooth surface, in just 2 weeks. Cumulatively, these studies demonstrate that the hydrogel formulation or bioengineered formulation of the present disclosure acts as a 'bio-instructive' scaffold for scar-less wound healing of cornea.

### EXAMPLE 5

### COMBINATION OF THE BIOENGINEERED FORMULATION AND EXOSOMES/SECRETOMES

The hydrogel formulation as described in the previous examples serve as an encapsulation scaffold that helps in promoting the sustained release of exosomes over a longer period of time when compared to direct application of exosomes/saline. The presence of exosomes in the hydrogel formulation broaden the scope of application of the said hydrogel formulations to treat severe corneal injuries and diseases such as anterior corneal scarring involving epithelial and stromal injuries/infection (active inflammation), stage 1 neurotrophic keratitis (NK) (persistent corneal epithelial defect), stage 2 NK (large persistent epithelial defect characterized by smooth, rolled edges), stage 3 NK (deep corneal ulcer, stromal melting, and sterile hypopyon), corneal ulcers such as Mooren's ulcer, Keratoconus and Corneal perforations. The combination product (hydrogel formulation + exosomes) helps in enhancing the wound healing efficacy of the hydrogel components with the addition of MSC-derived exosomes/secretome and cGMP grade stem cells.

The therapeutic effects of MSCs have been largely attributed to paracrine factors secreted by the cells including exosomes. Exosomes are nanometer-sized membrane-bound extracellular vesicles that act as mediators of crosstalk between cells. MSC-derived exosomes contain proteins such as growth factors, cytokines, lipid moieties and nucleic acids including miRNA and other non-coding RNAs (ncRNA). Some of the exosome associated proteins, typically known for their therapeutic applications, include MSC exosomes that are found to activate several signalling pathways important in wound healing (Akt, ERK, and STAT3). They also induce the expression of numerous growth factors, including hepatocyte growth factor (HGF), vascular endothelial growth factor (VEGF), insulin-like growth factor-1 (IGF1), nerve growth factor (NGF), stromal-derived growth factor-1 (SDF1), epidermal growth factor (EGF), and fibroblast growth factor (FGF), phosphoglycerate kinase (PGK), phosphoglucomutase, enolase, sFLT1 and miRNAs that include miR-10b, miR-21, miR-23a, miR-182, miR-181a, miR 145, and miR-205.

The present disclosure provides following combinations of the bioengineered formulation and the stem cell derived-exosomes:
Working example 1: Bioengineered formulation a: 33 kDa HA-MA (33 kDa)/RCP-SH (50 kDa) (20-75/20-250, mg/ml, DoS 50%) + 0.5-25 billion BMMSC-derived Exosomes/ml
Working example 2: Bioengineered formulation b: 33 kDa HA-MA/ 50 kDa RCP-SH (20-75/20-250, mg/ml, DoS 50%) + 0.5-25 billion CLSC-Exosomes/ml
Working example 3: Bioengineered formulation c: 33kDa HA-MA (33 kDa)/RCP-SH (50 kDa) (20-75/20-250, mg/ml, DoS 50%) + 0.5-25 billion CLSC-CM primed BMMSC-Exosomes/ml. Methodology:

### (a) Xenofree isolation and culture of corneal limbal stem cells (CLSCs), BMMSCs:

The xenofree protocol for the isolation and culture of CLSCs, BMMSCs, CLSC-CM primed BMMSC from human donors is described in the pending applications PCT/IN2020/050622, and PCT/IN2020/050623 which are incorporated in its entirety in the present disclosure.

### (b) Process of obtaining CLSC-CM or CSSC -CM primed BMMSC:

The priming protocol is described in the pending applications PCT/IN2020/050622, and PCT/IN2020/050623 which are incorporated in its entirety in the present disclosure.

### (c) Protocol for purification of exosomes & secretome from bone marrow-mesenchymal stem cells BMMSCs, CLSC, using iodixanol density gradient ultracentrifugation:

Protocol for purification of exosomes and secretomes is described in the pending applications PCT/IN2020/050622, and PCT/IN2020/050623 which are incorporated in its entirety in the present disclosure.

### (d) Protocol for purification of exosomes & secretomes from CLSC-primed BM-MSCs using iodixanol density gradient ultracentrifugation:

Protocol for purification of exosomes and secretomes from CLSC-primed BM-MSCs using iodixanol density gradient ultracentrifugation is described in the pending applications PCT/IN2020/050622, and PCT/IN2020/050623 which are incorporated in its entirety in the present disclosure.

The choice of purification protocol would be target indication and tissue specific. For example, the combination of BMMSCs + exosome purification protocol of Iodixanol gradient ultracentrifugation followed by size exclusion chromatography using Captocore700 column, is used for application in avascular tissues such as cornea since this combination would yield least quantities of angiogenic factors contaminating the exosome preparation (as described in the pending applications PCT Application No.: PCT/IN2020/050622 & PCT/IN2020/050623 which are incorporated in its entirety in the present disclosure). Capto Core 700 is composed of a ligand-activated core and inactive shell. The inactive shell excludes large molecules (cut off ~ Mr 700 000) from entering the core through the pores of the shell. These larger molecules are collected in the column flow through while smaller impurities bind to the internalized ligands. Furthermore, the resin Captocore700 is scalable to a capacity in litres. Exosomes of different purities will be developed for target indication specificity. For example, a combination of iodixanol density gradient ultracentrifugation or 30% sucrose cushion + Captocore700 would give us highest purity with minimal contamination with angiogenic factors (e.g. VEGF) that would be ideal for application in avascular tissues such as cornea (as described in the pending applications PCT Application No.: PCT/IN2020/050622 & PCT/IN2020/050623 which are incorporated in its entirety in the present disclosure).

### (e) Scalable culture of MSCs and CLSCs on microcarriers:

3D culture protocol is described in the pending applications PCT Application No.: PCT/IN2020/050622 & PCT/IN2020/050623 which are incorporated in its entirety in the present disclosure.

The process of purifying exosomes from human bone marrow derived stem cells (BM-MSC), human corneal limbal stem cells (CLSC) and CLSC-conditioned media (CLSC-CM) primed BM-MSCs (CLSC-CM/BM-MSC) has desirable regenerative potential. The detailed the secretory profile of CLSCs and CLSC-CM secretome and application thereof in multiple diseases including corneal ulcers and inflammatory conditions is provided in the pending applications PCT/IN2020/050622, and PCT/IN2020/050623 which are incorporated in its entirety in the present disclosure. The functional significance of increased HGF expression and reduced VEGF expression in CLSCs and CLSC-CM primed BMMSCs (provided in the pending applications PCT/IN2020/050622, and PCT/IN2020/050623 which are incorporated in its entirety in the present disclosure) is demonstrated in the present disclosure with respect to corneal applications.

Further, BMMSC-derived exosomes prepared by the process as described in the pending applications PCT/IN2020/050622, and PCT/IN2020/050623 which are incorporated in its entirety in the present disclosure neither promote nor inhibit angiogenesis. To further support this conclusion, the results provided in the pending applications PCT/IN2020/050622, and PCT/IN2020/050623 in its entirety in the present disclosure demonstrates that the exosome purification protocols (density gradient ultracentrifugation followed by size exclusion chromatography (Captocore 700) provided in the said pending applications, contain very low levels of VEGF. Hence, it can be contemplated that the exosomes derived from CLSC- and exosomes derived from CLSC-CM primed BMMSC prepared using the same protocols (as described in the pending applications PCT/IN2020/050622, and PCT/IN2020/050623 in its entirety in the present disclosure) will not exhibit any pro-angiogenic activity as well.

### Importance of Priming stem cells

Priming hBM-MSCs with CLSC-CM skew the phenotype of BM-MSCs towards a more CLSC-like profile. This helps to circumvent the need to isolate fresh CLSCs from human donor corneas, which are difficult to procure and also minimize donor to donor variation in exosome batch production. In addition, the yield of CLSCs is also very poor, when compared to commercially available sources of BM-MSCs. Hence, the process of reprogramming BM-MSCs to behave like CLSCs provide sufficient cell yields for the production of therapeutic exosomes. The results presented in the pending applications PCT/IN2020/050622, and PCT/IN2020/050623 demonstrates that priming BMMSCs with CLSC-CM increases the secretion of HGF and reduces the levels of VEGF and IL-6 in BMMSCs. In the present disclosure, about 0.5-1M stem cells (CLSC) were isolated per donor cornea that can be expanded to 4-6M in 3 passages. Commercially available BMMSCs can be expanded from 1M to 80-120M in 3 passages (RoosterBio Inc.). It is noteworthy to mention here that about 20-30 folds higher cell yield was achieved by using BMMSCs versus CLSCs. However, CLSCs (cornea resident MSCs) have shown to be immensely effective in corneal wound healing that cannot be mimicked by the use of BMMSCs. Therefore, in the present disclosure, BMMSCs were primed with CLSC-conditioned media to reprogram BMMSCs into CLSC-like stem cells. The process of priming BMMSCs with CLSC-conditioned media help to produce 20-60 folds higher CLSC-like BMMSC cell yield and exosomes. While using CLSC-exosomes can help treat 8-10 corneas at a dose of 0.1-0.5 billion exosomes per eye, whereas, the exosomes derived from CLSC-CM primed BMMSC helps to treat 20-60X i.e. 200-600 patients from a single donor cornea. Furthermore, by employing the 3D scalable cell expansion, the cell and exosome yield was amplified by an additional 5-10 folds. Hence, the combination of CLSC-CM priming process with 3D expansion methods yield 100-600 folds higher exosomes yield, thereby, allowing the treatment of approximately 1000-5000 patients per donor cornea (also described in the pending applications PCT/IN2020/050622, and PCT/IN2020/050623 which are incorporated in its entirety in the present disclosure).

### PROCESS FOR OBTAINING THE BIOENGINNERED FORMULATION COMPRISING EXOSOMES AND STEM CELLS

The process for obtaining the bioengineered formulation or hydrogel formulation comprising 33kDA HA-MA (33 kDa)/RCP-SH (50 kDa) and exosomes (derived from CLSC, BMMSCs, CLSCS-CM primed BMMSCs) is provided below:
(i) Vial I containing the lyophilized bioengineered cornea powder (comprising a combination of 33 kDa HA-MA having a concentration in the range of 2-7.5mg and RCP-SH having a concentration in the range of 5-15 mg/ml) and vial II containing the photoinitiator solution (100µl) was warmed to a root temperature RT protected from light.
(ii) Using a needle and syringe, the photoinitiator solution from vial II was transferred to the contents of vial I protected from light. Vial I was then gently swirled to ensure the contents are soaked to promote dissolution.
(iii) After the components of vial I were fully dissolved, lyophilized exosomes and cells were mixed with the final pre-hydrogel formulation to attain a homogenous solution.
(iv) Using a needle and syringe, the required volume was administered on to the cornea defect site and the pre-hydrogel formulation was exposed to white light for 5 min.
(v) Post-exposure to white light, the defect site was irrigated with saline solution to hydrate the crosslinked hydrogel.

The schematic representation of the process for obtaining the hydrogel formulation comprising stem cells and exosomes is depicted in Figure 17.

### RESULTS

Exosomes were purified and characterized according the process as described in the pending applications PCT/IN2020/050622, and PCT/IN2020/050623 which are incorporated in its entirety in the present disclosure.

### (A) Working example 1: Bioengineered formulation a: HA-MA (33 kDa)/RCP-SH (50 kDa) (20-75/20-250, mg/ml, DoS 50%) + 0.5-25 billion BMMSC-derived Exosomes/ml

Exosomes were purified and characterized according the process as described in the pending applications PCT/IN2020/050622, and PCT/IN2020/050623 which are incorporated in its entirety in the present disclosure.

### (i) Characterization of the anti-inflammatory activity of BM-MSC derived exosomes & CLSC-derived conditioned media

RAW 264.7 macrophage cells were seeded in 12 well plates and pre-treated either with 4 x 10⁸ exosomes (1µg) or conditioned media from CLSCs (25%, 50% substitution) (as indicated with grey color in Figure 18) overnight (16 h) followed by lipopolysaccharide (LPS) stimulation for 4 h. The cells were then washed and lysed for RNA extraction and quantification. The transcript levels of LPS induced cytokines such as IL-6, IFNy, TNF-α, IL-1β, IL-10 and VEGFA were quantitatively measured by real time RT-PCR. Human bone marrow-derived mesenchymal stem cells (hBM-MSC)-derived exosomes (fraction 9) and captocore purified sub-fraction F9-CC (fractions F9-2&3 pooled) significantly blocked inflammatory responses by macrophages in response to LPS stimulation (Figure 18). However, hBM-MSC derived exosomes did not have any significant effect on VEGFA transcript levels. Conditioned media from CLSCs also inhibited inflammatory cytokine expression in a dose-dependent manner (25% and 50% substitution of complete media with CLSC-derived conditioned media).

The secretory protein levels of cytokines were also measured in the supernatant collected from RAW 264.7 cells treated with BMMSC-exosomes to complement the transcript expression data shown in Figure 18 (A-F). The protein levels of IL-6, IL-1beta, TNF-alpha and IFN-gamma (as shown in Figure 18 G-J, respectively) were also significantly suppressed by BMMSC exosomes, confirming that the inhibitory effect of BMMSC exosomes occurred at the transcriptional level and was reflected in the final protein expression as well.

It can be inferred from Figure 18 that human BMMSC-derived exosomes and conditioned media derived from CLSCs inhibit the expression of inflammatory cytokines in macrophages.

### (ii) Characterization of the angiogenic activity of BM-MSC-derived exosomes

### (a) Anti-Angiogenesis activity of hBM-MSC-derived exosomes:

Coronary artery endothelial cells (CAECs) were seeded in serum-free growth media on growth factor reduced Matrigel, in VEGF supplemented media +/either with 4 x 10⁸ exosomes (1µg) for 24 h. Cells were stained with Cell Tracker^{™} Green CMFDA. As shown in Figure 19, no significant differences in tube formation were observed between control (Figure 19 A-B) and hBM-MSC-derived exosome (Figure 19 C-D) treated endothelial cells.

### (b) Pro-Angiogenesis activity of hBM-MSC-derived exosomes:

CAECs were seeded in serum-free growth media (no supplements) on Growth factor reduced matrigel +/- with 4 x 10⁸ exosomes (1 µg) for 24 h. Cells were stained with Cell Tracker ^{™} Green CMFDA. As demonstrated in Figure 19, no significant differences were observed in tube formation between control (Figure 19 E-F) and hBM-MSC-derived exosome (Figure 19 G-H) treated endothelial cells.

### (iii) Wound healing effect of hBM-MSC derived exosomes

The therapeutic functions of purified exosomes were characterized by determining the efficacy of wound healing exhibited by corneal epithelial cells in the presence or absence of exosomes in a 2D monolayer format. The cells were seeded on a flat surface and a scratch (mimicking a wound) was created across the monolayer.

Figure 20 depicts functional characterization of exosomes isolated from hBM-MSCs. Figure 20A represents images depicting the time course of the wound closure (2D scratch assay) on a monolayer of human corneal epithelial cells, observed across multiple time points (0, 12, 24, 48, 72 h). Figure 20B shows quantification of percentage of wound closure (2D scratch assay) in a monolayer of human corneal epithelial cells at 72 h.

It can be observed from Figure 20 that exosomes in F9 and F9-CC (pooled subfractions F9-2&3 from captocore purification step) showed superior wound healing capacity, when compared to vehicle alone control.

### (iv) Working example of influence of exosomes on viability of encapsulated cells and exosomes release assay (Bioengineered formulation (33 kDa HA-MA/ 50 kDa RCP-SH (20-75/20-250, mg/ml, DoS 50%) + stem cells + Exosomes)

To evaluate the influence of exosomes on cell viability of MSCs encapsulated in HA-MA/RCP-SH hydrogel formulation (comprising 30mg/ml of HA-MA, and 125 mg/ml), MSCs were encapsulated in the presence of exosomes, either supplemented in culture medium or encapsulated along with cells inside the hydrogels. Cell viability was evaluated using CCK8 assay at specific time points. Figure 22 shows the comparison between the cell activity of MSCs, combination of MSCs + exosomes in conditioned medium, and MSCs encapsulated in the hydrogel formulation of the present disclosure, in presence of exosomes. It can be contemplated that the exosomes herein included, but not limited to, exosomes derived from CLSC, or exosomes derived from CLSC-conditioned medium, or exosomes derived from CLSC-conditioned medium primed MSCs.

It can be observed from Figure 22 that in the presence of exosomes, cell viability of encapsulated MSCs (encapsulated in the hydrogel formulation of the present disclosure) was considerably higher compared to MSCs only (control). This result demonstrates that exosomes have the capacity to prolong the viability of cells encapsulated inside hydrogel matrices (Figure 22). Additionally, it can be observed from Figure 21 that exosomes encapsulated in the hydrogel formulation of the present disclosure were released into the surrounding media steadily with detectable levels being quantified from day 16 onwards.

Figure 22 depicts the influence of the exosomes on the cell viability of the MSCs encapsulated in the hydrogel formulation. It can be observed from Figure 22, that in the presence of exosomes (2.8x10⁹ particles/hydrogel supplemented in culture medium or encapsulated in hydrogel), cell viability of encapsulated MSCs (20000 cells/20 µl gel) was considerably higher for HA/RCP (40/125 mg/ml, DoS 50%) hydrogel formulation of the present disclosure, and Gel-MA (10% w/v, DoS 80%) hydrogels on day 5 compared to cell only control. G+C refers to Hydrogel with cells, GC Ex.CM refers to cell encapsulated gels receiving exosomes via culture medium, GC Ex.Enc. refers to cells and exosomes encapsulated together in gels. Data is represented as mean±SE with n=3 replicates.

It can be inferred form Figure 22 that the presence of exosomes helps in enhancing the cell viability of MSCs encapsulated in the hydrogel formulation of the present disclosure. The highest cell viability can be observed for the hydrogel of the present disclosure comprising cells and exosomes encapsulated together in the hydrogel. It was observed to be even higher than the Gel-MA, and as discussed previously, Gel-MA does not provide desirable results with respect to other parameters.

### (v) Human Dermal fibroblasts cultured on top of HA-MA/RCP-SH in the presence of exosomes

Human dermal fibroblasts (5x10⁵) were seeded on top of the hydrogel (40/125) in culture medium either supplemented with 4x10⁸ BMMSC exosomes or PBS control (Figure 23).

Figure 23A depicts culturing of human dermal fibroblasts in 2D culture; Figure 23B depicts culturing of human dermal fibroblasts on the surface of HA-MA/RCP-SH hydrogel formulation (comprising 40mg/ml of 33 kDa HA-MA and 125mg/ml of 50 kDa RCP-SH) and Figure 23C depicts culturing of human dermal fibroblasts on the surface of HA-MA/RCP-SH formulation (comprising 40mg/ml of 33 kDa HA-MA and 125mg/ml of 50 kDa RCP-SH) with exosome supplemented media. It can be observed from Figure 23 that the presence of exosomes in the hydrogel formulation of the present disclosure, did not appear to have any cytotoxic effect on the cells at the concentration used across all studies (0.4 billion exosomes/ml).

### (B) Working Example 2 and 3: Bioengineered formulation a: HA-MA (33 kDa)/RCP-SH (50 kDa) (20-75/20-250, mg/ml, DoS 50%) + 0.5-25 billion CLSC-derived Exosomes/ml or CLSC-CM primed BMMSC-derived exosomes/ml

### (i) Anti-inflammatory activity of CLSC-derived exosomes and CLSC-CM primed BMMSC- derived exosomes

The inflammatory profile of CLSC and CLSC-CM primed BMMSCs were interestingly different from BMMSCs. CLSC-derived exosomes and CLSC-CM primed BMMSC-derived exosomes significantly suppressed pro-inflammatory cytokine secretion in activated RAW 264.7 macrophages. However, the effect of BMMSC-derived exosomes was more than the CLSC-lineage exosomes (Figure 24).

Moreover, there was enhanced secretion of HGF in CLSC-CM primed BMMSCs, when compared to naive BMMSCs (results are described in the pending applications PCT/IN2020/050622, and PCT/IN2020/050623 which are incorporated in its entirety in the present disclosure). Additionally, CLSC-CM primed BMMSCs also demonstrated reduced VEGF secretion, resulting in a less angiogenic profile, common to parent CLSCs (results are described in the pending applications PCT/IN2020/050622, and PCT/IN2020/050623 which are incorporated in its entirety in the present disclosure). Hence, the combination of the anti-inflammatory activity and a HGF^{high}/VEGF^{low} profile maintained by CLSC- CM primed BMMSC exosomes leads to the exosomal product (bioengineered formulation + CLSC- CM primed BMMSC exosomes) of the present disclosure, customized for corneal defects.

### (C) Functional characterization of CLSC-conditioned media-derived secretome/exosomes and CLSC-CM primed BMMSC conditioned media-derived secretome/exosomes

### (i) Angiogenesis activity of CLSC-Conditioned media/secretome

CAECs were seeded in serum-free growth media on growth factor reduced matrigel with/without CLSC-conditioned media (CM) for 24 h. Cells were stained with Cell Tracker^{™} Green CMFDA. Figure 25 shows the angiogenesis activity of CLSC-CM/secretome. It can be observed from Figure 25 that CLSC-CM/secretome significantly disrupted the tube formation ability of the endothelial cells, when compared to the vehicle (PBS; control) only in a dose dependent manner.

### (ii) Anti-inflammatory activity of CLSC-conditioned media-derived secretome and CLSC-CM primed BMMSC conditioned media-derived secretome

RAW 264.7 macrophages were activated with LPS in the presence or absence of conditioned media-derived secretome collected from BMMSCs, CLSCs or CLSC-CM primed BMMSCs (10% and 25%). The cells were maintained in 50% conditioned media supplemented growth media overnight and activated with LPS for 4 hours.

Figure 26 shows anti-inflammatory effect of CLSC-conditioned media-derived secretome and CLSC-CM primed BMMSC conditioned media-derived secretome on RAW 264.7 macrophage cells. RAW 264.7 macrophage cells were treated either with conditioned media collected from CLSCs and CLSC-CM primed BMMSCs at 50% supplementation (collected from 0.5 million BMMSCs) followed by LPS stimulation for 4 h. The levels of secreted cytokines were also quantified by ELISA for (A) IL-6, (B) IL-10, (C) TNF-α and (D) IL-1β.

It can be observed from Figure 26 that secretome from all three cell types (BMMSCs, CLSCs or CLSC-CM primed BMMSCs) showed comparable anti-inflammatory activity.

### (iii) Wound healing activity of BMMSC conditioned media, CLSC-conditioned media-derived secretome and CLSC-CM primed BMMSC conditioned media-derived secretome

The therapeutic functions of secretomes collected from BMMSC, CLSC and CLSC-CM primed BMMSCs (25% & 10%) were characterized by determining the efficacy of wound healing exhibited by corneal epithelial cells in the presence or absence of exosomes in a 2D monolayer format. The cells were seeded on a flat surface and a scratch (mimicking a wound) was created across the monolayer.

Figure 27 shows that comparison of the wound healing activity of CLSCs, CLSC-CM primed BMMSCs, and BMMSC secretome. (A) Representative images depicting the time course of the wound closure (2D scratch assay) on a monolayer of human corneal epithelial cells in the presence of secretome (equivalent to 0.2 million source MSCs), observed across multiple time points (0, 12, 24, 48 h); (B) Representative images depicting the time course of the wound closure (2D scratch assay) on a monolayer of human corneal epithelial cells in the presence of 4 x 108 exosomes (equivalent to 0.2 million source MSCs), observed across multiple time points (0, 12, 24, 48,72 h).

Figure 27 clearly demonstrates that CLSC-derived secretome and CLSC-CM primed BMMSC-derived secretome were more superior in promoting the migration of human corneal epithelial cells in the wound healing assay, when compared to the BMMSC-derived secretome. Therefore, it can be inferred that CLSC-derived secretome and CLSC-CM primed BMMSC-derived secretome exhibits superior wound healing capacity as compared to naive BMMSC- derived secretome.

### (iv) Superior reinnervation activity

PC12 is a suspension cell line derived from a pheochromocytoma of the rat adrenal medulla that has an embryonic origin from the neural crest. These cells have been well established to acquire neuronal phenotype when activated with NGF. 5x104/ml cells were seeded on collagen coated wells and allowed to adhere overnight. The cells were either treated with NGF (positive control) (20ng/ml) or indicated exosomes samples (doses mentioned in figure legend) and live imaged at 24h. Figure 28 shows the results of *in-vitro* innervation assay. It can be observed from Figure 28 that CSSC-derived exosomes and CSSC-CM primed BMMSC-derived exosomes promoted innervation, as demonstrated by the induction of neurite outgrowth in an *in-vitro* innervation assay (Figure 28 C-E). In contrast, no neurite outgrowth formation was reported in PBS (Figure 28A) and BMMSC exosomes treated cells (Figure 28F respectively). Recombinant human Nerve Growth Factor (rhNGF) (20ng/ml) was included as a positive control (Figure 28B). NGF is a standard neuron activation/differentiation stimulus commonly used in innervation assays.

Further to validate the results of the innervation assay, the protein levels of NGF secreted by CLSC-CM primed BMMSCs, in both the secretome and in exosomes, were assessed by ELISA. Figure 29 shows the secretory levels of NGF. Referring to Figure 29, CLSC-CM primed BMMSCs secreted NGF in the exosomes (Figure 29A) and secretome (Figure 29B) and albeit at concentrations lower than CLSCs. Analysis of the secretome by western blot also confirmed this trend (Figure 29C).

Therefore, it can be observed from Figure 28 and Figure 29, that CLSC-derived exosomes and CLSC-CM primed BMMSC-derived exosomes promotes innervation (neurite outgrowth) in PC12 cells at 0.4 billion exosomes/ml.

### (v) Superior anti-fibrosis activity of CSSC- and CSSC-CM primed BMMSC exosomes

Figure 30 shows anti-fibrotic effect of CLSC-derived exosomes and CLSC-CM primed-derived exosomes. Human dermal fibroblasts were pre-treated with indicated exosomes (4x10⁸/ml) for 4 hours prior to induction of fibrosis with TGF-β (10ng/ml) for 24 hours. Cells were stained with anti-α-SMA antibody (as indicated with green color) (a marker of fibrosis) and DAPI (nucleus, indicated with blue color).

It can be observed from Figure 30 that fibrosis (α -SMA expression) was induced in Human Dermal fibroblasts with TGF- β (10ng/ml) in the presence of CLSC-derived exosome, CSSC-CM primed BMMSC-derived exosomes or BMMSC-derived exosomes. CLSC- and CLSC-CM primed BMMSC -derived exosomes inhibited TGF- β induced expression of α -SMA (Figure 30C-E) while BMMSC-derived exosomes (Donor 200) had very little effect on inhibiting the induction of α -SMA (Figure 30F). Both 10% CLSC-CM and 25% CLSC-CM priming inhibited α -SMA expression in human dermal fibroblasts in a dose dependent manner (Figure 30D-E).

### (vi) Characterization of angiogenic activity of CLSC and CLSC-CM primed -derived exosomes and secretome.

Figure 31 shows the characterisation of angiogenic activity of CLSC and CLSC-CM primed BMMSC-derived exosomes and secretome. It can be observed from Figure 31A and 31B that CLSCs expressed less VEGF in their secretome and exosomes, as compared to BMMSCs. Further, it can be observed from Figure 31C to Figure 31E that CLSCs expressed more sFLT1 (VEGFR1) in their secretome and Exosomes than BMMSCs. sFLT1 has been reported to be secreted by corneal stem cells and have been shown to be responsible for the avasculature structure of cornea. Therefore, it can be inferred from Figure 31 that priming of BMMSCs with CLSC-CM could reduce the angiogenic potential of BMMSCs by reducing the VEGF secretion and increasing the sFLT1 levels in the secretome and exosomes.

It can be contemplated that the above results also apply when the stem cells, or exosomes, or in combination thereof are encapsulated in the hydrogel formulation of the present disclosure. Therefore, the hydrogel formulation of the present disclosure is biocompatible and exhibits cornea-mimetic properties. Therefore, it can be concluded that the presence of the modified collagen peptide (RCP-SH/ RCP-MA) with a molecular weight in the range of 20-80 kDa, and with a degree of substitution in the range of 20-75%; and the modified hyaluronic acid (HA-MA) with molecular weight in the range of 10-100 kDa, and with a degree of substitution in the range of 20-75% is essential to arrive at the bioengineered formulation or hydrogel formulation of the present disclosure, that not only mimics the properties of the native cornea, but can also be applied at the site of corneal defect to treat various corneal disorders. Moreover, the presence of stem cells, or exosomes, or combinations thereof in the hydrogel formulation of the present disclosure helps in enhancing the cell viability and cell proliferation, confirming biocompatibility and cornea-mimetic properties of the hydrogel formulation. The data presented in Figure 21 and 22 shows the proof of concept that the exosomes perform well when present in the hydrogel formulations. Therefore, the properties of the exosomes as shown in the present Example will also hold good when the exosomes are present in the hydrogel formulation (bioengineered formulation) of the present disclosure.

The examples provided in the present disclosure provides the hydrogel formulation comprising a modified hyaluronic acid (HA-MA) with molecular weight of "10kDa, or "33 kDa", or "50 kDa" and with concentration of 35 mg/ml, 40 mg/ml, 75 mg/ml and a modified collagen peptide (RCP-SH) with molecular weight of "50 kDa", and with concentration of 125 mg/ml,150 mg/ml. However, it can be contemplated that a person skilled in the art can arrive at the hydrogel formulation of the present disclosure that shows desired physical and functional properties, by using combinations of HA-MA/RCP-SH with different molecular weight, i.e., 20/30 kDa, or 40/55 kDa, or 50/60 kDa, or 80/70 kDa, or 90/80 kDa, and with concentrations of 20/125 mg/ml, or 25/125mg/ml, or 30/125mg/ml, or 50/125mg/ml, or at any other disclosed ranges as described in the present disclosure. Further, encapsulation of stem cells, or exosomes, or combination thereof, incorporated in the hydrogel formulation having different combinations of HA-MA/ RCP-SH at the aforementioned concentrations or the disclosed ranges, would also produce the similar results as exemplified herein.

### EXAMPLE 6

### EYE DROP FORMULATION

The present disclosure also provides eye drop formulation. The eye drop formulation comprises: (a) exosomes selected from the group consisting of corneal stromal stem cell derived-exosomes, primed mesenchymal stem cell derived-exosomes, and naive mesenchymal stem cell derived-exosomes; and (b) a clinically approved eye drop formulation. The eye drop formulation helps in treating corneal injuries and ulcers. The eye drop formulation is administered as a standalone treatment option or as an adjuvant treatment option to patients receiving the hydrogel formulation of the present disclosure. The dosage will be as recommended and prescribed by the clinician. Some of the clinically approved eye drop formulation that can be used for topical application of the exosomes of the present disclosure includes: (a) Tearhyl^{®} (Sodium hyaluronate, 0.1-0.3% solution); (b) Refresh Optive^{®} (Carboxymethylcellulose, 0.5% solution); (c) Systane Ultra^{®} (Polyethylene glycol, MW 400, 0.4% solution); (d) Leader^{®} Artificial Tears Solution (Polyvinyl alcohol, 1.4% solution); (e) Systane Balance^{®} (Propylene glycol, 0.6% solution); and (f) MIKELAN^{®} LA (Alginate based).

In the present disclosure, the exosomes (corneal stromal stem cell derived-exosomes, primed mesenchymal stem cell derived-exosomes, and naive mesenchymal stem cell derived-exosomes) were administered in two indication-specific approaches:
(i) Eye drop formulation: The eye drop formulation as discussed above helps in assisting/enhancing the therapeutic effects of chosen standard of care. They will be administered for indications listed below: (a) Superficial Corneal surface abrasions; (b) Corneal epithelial injuries; (c) Stage 1 neurotrophic keratitis (NK): persistent corneal epithelial defect; (d) Dry eyes.
(ii) Exosomes encapsulated in liquid cornea hydrogel followed by post-operative care using exosomal eye drops (post hydrogel application): Sustained release of exosomes over a period of time not only enhance efficient re-epithelialization but also promote resolution of injury-induced fibrosis and inflammation surrounding the injury. The combination of encapsulated exosomes with exosomal eye drops allow suppression of any inflammatory responses and gradual healing of fibrotic scars with no neovascularization. They will be administered for indications listed below: (a) Anterior corneal scarring involving epithelial and stromal injuries/infection (active inflammation); (b) Stage 1 neurotrophic keratitis (NK): persistent corneal epithelial defect; (c) Stage 2 NK: large persistent epithelial defect characterized by smooth, rolled edges; (d) Stage 3 NK: deep corneal ulcer, stromal melting, and sterile hypopyon; (e) Mooren's ulcer;

### (f) Keratoconus; and (g) Corneal perforations.

### Working example: Eye drop formulation containing 0.1-0.5% clinical grade Hyaluronic acid (HA) + 0.4 billion exosomes/ml of 25% CLSC-CM primed BMMSC exosomes.

### (i) Cellular uptake of eye drop formulation

Exosomes were labelled with PKH26 as per manufacturer's recommendations. Excess dye was removed by repeated ultracentrifugation at 100,000xg for 2 hours in PBS (50 times sample volume). The eye drop formulations comprising 0.1-5% HA and 4x10⁸ exosomes/ml were prepared fresh and added to human Corneal Epithelial Cells and incubated for 4 hours at 37C. Cells were imaged live (Figure 32) and post- fixation followed by immunostaining with Cytokeratin-3/DAPI (green/DAPI) (Figure 33). As shown in Figure 33, exosomes (red) were taken up by cells (green) even in the presence of varying concentrations of clinical grade HA, demonstrating the biocompatibility of HA with exosome cellular uptake. Uptake of exosomes was observed across all tested formulations. However, a decrease in exosome uptake was observed at higher HA concentrations of HA 1-5% (Figure 33E and 33F). Hence, the presence of clinical grade HA at a concentration (1-5%) higher than the disclosed concentration range (0.1-0.5%) serve as non-working examples. Therefore, it is important for the clinical grade HA to be present at a concentration in the range of 0.1-0.5% in order to demonstrate biocompatibility of HA with exosome cellular uptake.

### (ii) Anti-inflammatory activity of eyedrop formulations

The anti-inflammatory activity of CLSC-CM primed BMMSC-derived exosomes was quantified in order to evaluate the effect of HA on the exosomal activity. As shown in Figure 34 no significant difference was observed in the anti-inflammatory activity of exosomes in the presence of HA at various concentrations, thereby suggesting that HA had no adverse effects on the exosomal therapeutic activity in inhibiting inflammatory responses. Additionally, it was also observed that HA alone does not elicit any anti-inflammatory effects on macrophages, confirming that exosomes form the key therapeutic component of the eyedrop formulation.

### EXAMPLE 7

### METHOD OF TREATMENT OF CORNEAL DISORDER

The present disclosure also provides a method of treating the corneal disorder. The method of treating the corneal disorder comprises the steps of: (a) the bioengineered formulation of the present disclosure was obtained; (b) a suitable amount of the bioengineered formulation was applied at the site of a corneal defect; and (c) a white light having an intensity in the range of 50-150mW/cm² was illuminated on the formulation at the site of the corneal defect for a time period in a range of 1-15 minutes, preferably, 2-8 minutes, for treating the corneal disorder in a subject. The presence of the stem cells, or exosomes, or combinations thereof in the bioengineered formulation helps in enhancing the wound healing capacity of the bioengineered formulation.

Further, the present disclosure also provides another method of treating a corneal disorder in a subject. The method comprises the steps of : (a) the eye drop formulation comprising: (i) exosomes selected from the group consisting of corneal stromal stem cell derived-exosomes, primed mesenchymal stem cell derived-exosomes, and naive mesenchymal stem cell derived-exosomes was obtained; and (ii) a clinically approved eye drop formulation; and (b) the eye drop formulation was applied at the site of the corneal defect, for treating the corneal defect in a subject. The sustained release of exosomes over a period of time not only enhance efficient re-epithelialization but also promote resolution of injury-induced fibrosis and inflammation surrounding the injury. The combination of encapsulated exosomes with eyedrop formulation allow suppression of any inflammatory responses and gradual healing of fibrotic scars with no neovascularization.

### EXAMPLE 8

### CALCULATION OF MOLECULAR WEIGHT BASED ON INTRINSIC VISCOSITY

Figure 35 depicts representative raw data obtained from rheometer for the calculation of intrinsic viscosity. Intrinsic viscosity is defined as the viscosity at shear rate approaching 0.

The intrinsic viscosity can be correlated with molar mass using the Mark-Houwink equation (https://wiki.anton-paar.com/ en/intrinsic-viscosity-determination/)

I.V. = K (M^a), where K, a are Mark-Houwink constants.

For HA MW of "33 kDa", the constants K and a are 0.036 and 0.78 respectively. (Practical aspects of Hyaluronan Based Medical Products" by J.W Kuo, Page 83).

Based on these values the MW of "33 kDa" HA-MA is calculated to be ~12 kDa.

Table 3 shows molecular weight estimations of HA and HA-MA derivative based on different techniques.

**Table 3**

| **Material/Source** | **Molecular weight (kDa)** | | | |
|---|---|---|---|---|
| | Based on supplier | | Based on the present disclosure | |
| | GPC | I.V. | GPC | I.V. |
| HA-MA (CreativePEG Works) | 10, 50 and 250 | - | - | - |
| HA (Stanford Chemicals) | - | 33 | 89 | ~46 |
| HA-MA (CreativePEG Works) | - | - | 44 | ~12 |

### Definition of "33 kDa" HA-methacrylate

As per Stanford Chemicals, the Molecular weight of Hyaluronic acid (HA) raw material was 33 kDa (Figure 36) based on their intrinsic viscosity (i.v.) measurements. However, based on the in-house GPC data the MW range for this HA raw material was found to be 48-148 kDa with peak MW of 89 kDa (Figure 36).

The "33 kDa" raw material was methacrylated by CreativePEG Works to yield HA-MA. The MW range according to in-house GPC was found to be 11-100 kDa with peak MW at 44 kDa (Figure 37).

Also, based on the calculation of Molecular weight based on intrinsic viscosity as provided in the present disclosure, the MW of "33 kDa" HA MA was found to ~12 kDa. Therefore, the term "33 kDa HA-MA" refers to the molecular weight of the molecule which was obtained commercially, and as a part of the study, the present disclosure also discloses the calculation of molecular weight to be approximately 12 kDa, and therefore, a range has been provided in the present disclosure. A person skilled in the art can procure the above-mentioned molecules commercially to perform the experiments.

### Calculation of degree of substitution from H-NMR data

Figure 38 shows the representative H-NMR data of "33 kDa" HA-MA. The degree of substitution (DoS) was determined by comparing the ratio of the areas under the proton peaks at around 5.6 and 6.1 ppm (methacrylate protons) to the peak at ~1.9 ppm (N-acetyl glucosamine of HA). (Seidlits, S.K., et al. The effects of hyaluronic acid hydrogels with tunable mechanical properties on neural progenitor cell differentiation. Biomaterials,31,3930-3940 (2010). https://doi.org/10.1016/j.biomaterials.2010.01.125)); Chandrasekharan A., et al. In situ photocrosslinkable hyaluronic acid-based surgical glue with tunable mechanical properties and high adhesive strength. J. Polym. Sci., Part A: Polym. Chem. 2019, 57, 522-530. https:// doi.org/10.1002 /pola.29290).

Therefore, DoS = [((Area under the peaks 5.8 ppm + Area under the peak at 6.25 ppm)/2)*3]/Area under the peak at 2.07 ppm X 100= [(4.057/2)*3]/3.807 X 100 = ~160%. Therefore, the mentioning of degree of substitution (DoS) as 50 % in the present disclosure is as per the information provided by the vendor.

### Advantages of the present disclosure

The present disclosure provides a bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%. Particularly, the present disclosure provides a bioengineered formulation comprising: (a) a modified collagen peptide having a molecular weight in the range of 30-60 kDa, and with a degree of substitution in the range of 35-55%; and (b) a modified hyaluronic acid having a molecular weight in the range of 10-48 kDa, and with a degree of substitution in the range of 33-55%, wherein the modified collagen peptide includes, but not limited to thiolated collagen peptide, and wherein the modified hyaluronic acid includes, but not limited to methacrylated collagen peptide. The modified collagen peptide is in the concentration range of 20-250 mg/ml with respect to the formulation, and wherein the modified hyaluronic acid is in the concentration range of 20-80 mg/ml with respect to the bioengineered formulation. Preferably, the modified collagen peptide is in the concentration range of 20-150 mg/ml with respect to the formulation, and wherein the modified hyaluronic acid is in the concentration range of 20-75 mg/ml. The present disclosure also provides a process for obtaining the bioengineered formulation. In the said process, photo-initiator solution is added to the bioengineered formulation which is followed by an exposure to a white light intensity in the range of 50-150mW/cm² for a time period in the range of 1-15 minutes, preferably, 2-8 minutes, that helps in obtaining a cross-linked hydrogel. The photo-initiator solution comprises 0.001-0.1 mM Eosin Y and 0.038% w/v triethanolamine in phosphate buffered saline solution. The present disclosure further comprises stem cells and/or exosomes, wherein the stem cells is selected from the group consisting of human bone marrow- mesenchymal stem cell, adipose tissue-mesenchymal stem cell, umbilical cord- mesenchymal stem cell, Wharton jelly-mesenchymal stem cell, dental pulp-derived mesenchymal stem cell, and corneal limbal stem cell-derived conditioned media primed mesenchymal stem cells, and wherein the exosomes is selected from the group consisting of corneal stromal stem cell derived-exosomes, primed mesenchymal stem cell derived-exosomes, and naive mesenchymal stem cell derived-exosomes. As shown in Table 1, the bioengineered formulation of the present disclosure has a compressive modulus in the range of 100-1400 kPa, preferably 100-500 kPa. Moreover, the bioengineered formulation is resistant to at most 50% degradation within 28 days under *in-vitro* conditions. The results of Table 1 show that the physical properties of the bioengineered formulation match with the characteristic properties of the native cornea, thereby demonstrating that the bioengineered formulation exhibits bio-mimetic properties. Further, as shown in Figure 16, the application of the bioengineered formulation in rabbit cornea wound under *in-vivo* conditions, showed gradual gain on transparency, no angiogenesis and completely heals the epithelium with smooth surface, in just 2 weeks. This shows that bioengineered formulation of the present disclosure acts as a 'bio-instructive' scaffold for scar-less wound healing of cornea. Further the present disclosure provides a formulation comprising: (a) exosomes selected from the group consisting of corneal stromal stem cell derived-exosomes, primed mesenchymal stem cell derived-exosomes, and naive mesenchymal stem cell derived-exosomes; and (b) a clinically approved eye drop formulation. Moreover, the method of treating a corneal defect is also provided herein. Also, the use of bioengineered formulation, or the formulation for treating a corneal defect is also provided herein.

Overall, the bioengineered formulation encompasses properties including anti-fibrotic, anti-angiogenic, anti-inflammatory and pro-reinnervation. Moreover, the bioengineered formulation of the present disclosure is a cross-linked hydrogel which has the desirable features of being bio-mimetic, bio-compatible, and biodegradable. The present disclosure also provides a convenient and time-efficient process for preparing the bioengineered formulation. The bioengineered formulation also promotes scar-less corneal healing, thereby, resulting in transparent cornea after performing the procedure using the bioengineered formulation as described in the present disclosure.

The bioengineered formulation of the present disclosure helps in treating corneal defect or corneal diseases, including but not limited to anterior corneal scarring involving epithelial and stromal injuries/infection (active inflammation), Stage 1 neurotrophic keratitis (NK) (persistent corneal epithelial defect), Stage 2 NK (large persistent epithelial defect characterized by smooth, rolled edges), Stage 3 NK (deep corneal ulcer, stromal melting, and sterile hypopyon), corneal ulcers such as Mooren's ulcer, Keratoconus and Corneal perforations. The bioengineered formulation of the present disclosure also helps in treating corneal limbal injuries and corneal dystrophies (CDs), such as lattice CD type 1, granular CD type 1, and congenital stromal CD, wherein the corneal stroma is damaged in the subject. Moreover, the bioengineered formulation of the present disclosure acts as potential treatment for Schnyder CD and lattice CD type-2, wherein both the epithelium and stroma are compromised. The use of the bioengineered formulation of the present disclosure is followed by post-operative care using exosomal eye drops (post hydrogel application) that allow sustained release of stem cells, or exosomes, or combinations thereof over a period of time, which not only enhances efficient re-epithelialization but also promotes resolution of injury-induced fibrosis and inflammation surrounding the injury. The combination of encapsulated exosomes and the formulation allows suppression of any inflammatory responses and gradual healing of fibrotic scars with no neovascularization.

The invention can be further defined with reference to the following embodiments or clauses:
1. A bioengineered formulation comprising: (a) a modified collagen peptide having a molecular weight in the range of 20-80 kDa, and with a degree of substitution in the range of 20-75%; and (b) a modified hyaluronic acid having a molecular weight in the range of 10-100 kDa, and with a degree of substitution in the range of 20-75%.
2. A bioengineered formulation comprising: (a) a first polymer selected from the group consisting of collagen peptide, modified collagen peptide, collagen, and modified collagen; and (b) a second polymer selected from the group consisting of hyaluronic acid, modified hyaluronic acid, cellulose, modified cellulose, polyethylene glycol, modified polyethylene glycol, polyvinyl alcohol, modified polyvinyl alcohol, poly(N-isopropylacrylamide), modified poly(N-isopropylacrylamide), silk, modified silk, gelatin, modified gelatin, alginate, and modified alginate, wherein the bioengineered formulation has a compressive modulus in the range of 100-1400 kPa, preferably 100-500 kPa.
3. The bioengineered formulation of clauses 1 or 2, wherein the modified hyaluronic acid having a molecular weight in the range of 10-48 kDa, preferably 12-35kDa.
4. The bioengineered formulation of clauses 1 or 2, wherein the modified collagen peptide is in the concentration range of 20-250 mg/ml with respect to the formulation, and wherein the modified hyaluronic acid is in the concentration range of 20-80 mg/ml with respect to the bioengineered formulation.
5. The bioengineered formulation of clauses 1 or 2, wherein the modified collagen peptide is selected from the group consisting of a thiolated collagen peptide, and a methacrylated collagen peptide.
6. The bioengineered formulation of clause 1 or 2, wherein the modified hyaluronic acid is selected from the group consisting of a methacrylated hyaluronic acid, and a thiolated hyaluronic acid.
7. The bioengineered formulation as in clause 1, wherein the formulation further comprises at least one type of stem cells selected from the group consisting of mesenchymal stem cells, corneal stromal stem cells, corneal limbal stem cells, and induced pluripotent stem cells.
8. The bioengineered formulation as in clause 7, wherein the mesenchymal stem cell is selected from the group consisting of human bone marrow-mesenchymal stem cell, adipose tissue- mesenchymal stem cell, umbilical cord- mesenchymal stem cell, Wharton jelly- mesenchymal stem cell, dental pulp-derived mesenchymal stem cell, and corneal limbal stem cell-derived conditioned media primed mesenchymal stem cells.
9. The bioengineered formulation as in clause 7, wherein the stem cells are present in the range of 0.1-10 million cells per ml of the bioengineered formulation.
10. The bioengineered formulation of clauses 1 or 2, wherein the bioengineered formulation further comprises exosomes selected from the group consisting of corneal stromal stem cell derived-exosomes, primed mesenchymal stem cell derived-exosomes, and naive mesenchymal stem cell derived-exosomes.
11. The bioengineered formulation as in clause 10, wherein the exosomes has a concentration in the range of 0.5-25 billion exosomes per ml of the bioengineered formulation.
12. The bioengineered formulation as in clause 10, wherein the primed mesenchymal stem cell derived-exosomes are exosomes derived from mesenchymal stem cells primed with corneal stromal stem cell derived-conditioned medium.
13. The bioengineered formulation of clauses 1 or 2, wherein the modified hyaluronic acid is methacrylated hyaluronic acid, and wherein the modified collagen is thiolated collagen peptide.
14. The bioengineered formulation of clauses 1 or 2, wherein the formulation further comprises: (i) stem cells selected from the group consisting of mesenchymal stem cells, corneal stromal stem cells, and corneal limbal stem cells; and (ii) exosomes selected from the group consisting of corneal stromal stem cell derived-exosomes, primed mesenchymal stem cell derived-exosomes, and naive mesenchymal stem cell derived-exosomes.
15. The bioengineered formulation of clauses 1 or 2, wherein the formulation has a compressive modulus in the range of 100-1400 kPa, preferably 100-500 kPa.
16. The bioengineered formulation of clauses 1 or 2, wherein the formulation is resistant to at most 50% degradation within 28 days under *in-vitro* conditions.
17. A process for obtaining the bioengineered formulation as in clause 1, said process comprising:
   (a) contacting the modified collagen peptide having a molecular weight in the range of 20-80 kDa, and with a degree of substitution in the range of 20-75% to the modified hyaluronic acid having a molecular weight in the range of 10-100 kDa, and with a degree of substitution in the range of 20-75%, to obtain a pre-mix; and
   (b) contacting the pre-mix with a photo-initiator solution, to obtain the bioengineered formulation.
18. The process as in clause 17, wherein the modified collagen peptide has a concentration in the range 20-250 mg/ml with respect to the formulation, and wherein the modified hyaluronic acid has a concentration in the range of 20-80 mg/ml with respect to the bioengineered formulation.
19. The process as in clause 17, wherein the photo-initiator solution comprises 0.001-0.1 mM Eosin Y and 0.038% w/v triethanolamine in phosphate buffered saline solution, and wherein the photo-initiator solution is present in an amount ranging from 0.5X-1X with respect to the bioengineered formulation.
20. The process as in clause 17, wherein contacting the pre-mix with the photo-initiator solution is followed by an exposure to a white light having an intensity in the range of 50-150mW/cm² for a time period in the range of 1-15 minutes, preferably, 2-8 minutes.
21. A method for treating a corneal defect in a subject, said method comprises:
   (a) obtaining the bioengineered formulation of clauses 1-16;
   (b) applying a suitable amount of the bioengineered formulation at the site of a corneal defect; and
   (c) illuminating a white light having an intensity in the range of 50-150mW/cm² on the formulation at the site of the corneal defect for a time period in a range of 1-15 minutes, preferably, 2-8 minutes, for treating the corneal defect in a subject.
22. The method as in clause 21, wherein the method further comprises applying a solution comprising: (i) exosomes selected from the group consisting of corneal stromal stem cell derived-exosomes, primed mesenchymal stem cell derived-exosomes, and naive mesenchymal stem cell derived-exosomes; and (ii) a clinically approved eye drop formulation, at the site of the corneal defect before or after applying the suitable amount of the formulation.
23. A formulation comprising: (a) exosomes selected from the group consisting of corneal stromal stem cell derived-exosomes, primed mesenchymal stem cell derived-exosomes, and naive mesenchymal stem cell derived-exosomes; and (b) a clinically approved eye drop formulation.
24. The formulation as in clause 23, wherein the clinically approved eye drop formulation comprises at least one polymer selected from the group consisting of hyaluronic acid, carboxymethyl cellulose, polyethylene glycol, polyvinyl alcohol, propylene glycol, and alginate.
25. A method for treating a corneal defect in a subject, said method comprising:
   (a) obtaining a formulation comprising: (i) exosomes selected from the group consisting of corneal stromal stem cell derived-exosomes, primed mesenchymal stem cell derived-exosomes, and naive mesenchymal stem cell derived-exosomes; and (ii) a clinically approved eye drop formulation; and
   (b) applying the formulation at the site of the corneal defect, for treating the corneal defect in a subject.
26. The bioengineered formulation of clauses 1-16, for use in treating a corneal defect in a subject.
27. The formulation as in clause 23 for use in treating a corneal defect in a subject.

## Claims

1. A formulation comprising: (a) exosomes selected from the group consisting of exosomes derived from corneal stromal stem cells, exosomes derived from primed mesenchymal stem cells, and exosomes derived from naive mesenchymal stem cells; and (b) a clinically approved eye drop formulation, wherein the clinically approved eye drop formulation comprises at least one polymer selected from the group consisting of hyaluronic acid, carboxymethyl cellulose, polyethylene glycol, polyvinyl alcohol, propylene glycol, and alginate.

2. The formulation of claim 1, wherein the primed mesenchymal stem cells are primed with corneal stromal stem cell derived-conditioned medium.

3. The formulation of claim 1 or claim 2, wherein the exosomes derived from the primed mesenchymal stem cells are **characterized by** having, when compared to exosomes derived from the naive mesenchymal stem cells:
a higher expression level of sFLT1; and
a lower expression level of vascular endothelial growth factor (VEGF).

4. The formulation of any one of claims 1-3, wherein the primed mesenchymal stem cell-derived exosomes are **characterized by** substantially lacking in VEGF expression.

5. The formulation of any one of claims 1-4, wherein the primed mesenchymal stem cells are primed bone marrow-derived mesenchymal stem cells.

6. The formulation of any one of claims 1-5, wherein the formulation further comprises at least one type of stem cells selected from the group consisting of mesenchymal stem cells, corneal stromal stem cells, corneal limbal stem cells, and induced pluripotent stem cells, and wherein, optionally, the mesenchymal stem cells are selected from the group consisting of bone marrow-derived mesenchymal stem cells, adipose tissue-derived mesenchymal stem cells, umbilical cord-derived mesenchymal stem cells, Wharton jelly-derived mesenchymal stem cells, dental pulp-derived mesenchymal stem cells, and corneal limbal stem cell-derived conditioned media primed mesenchymal stem cells.

7. The formulation of any one of claims 1-6, wherein the exosomes have a concentration in the range of 0.5-25 billion exosomes per ml of the bioengineered formulation.

8. The formulation of any one of claims 1-7 for use in treating a corneal defect in a subject.
